(19) **Europäisches Patentamt**
**European Patent Office**
**Office européen des brevets**

(11) **EP 1 896 127 B1**

(12) **EUROPEAN PATENT SPECIFICATION**

(45) Date of publication and mention
of the grant of the patent:
**29.05.2013 Bulletin 2013/22**

(51) Int Cl.:
***A61N 1/372*** *(2006.01)*

(21) Application number: **05742622.3**

(86) International application number:
**PCT/SE2005/000652**

(22) Date of filing: **04.05.2005**

(87) International publication number:
**WO 2006/118499 (09.11.2006 Gazette 2006/45)**

(54) **SYNCHRONIZATION OF IMPLANTABLE MEDICAL DEVICES**

SYNCHRONISIERUNG VON IMPLANTIERBAREN MEDIZINPRODUKTEN

SYNCHRONISATION DE DISPOSITIFS MEDICAUX IMPLANTABLES

(84) Designated Contracting States:
**AT BE BG CH CY CZ DE DK EE ES FI FR GB GR
HU IE IS IT LI LT LU MC NL PL PT RO SE SI SK TR**

(43) Date of publication of application:
**12.03.2008 Bulletin 2008/11**

(73) Proprietor: **St. Jude Medical AB
175 84 Järfälla (SE)**

(72) Inventor: **ABRAHAMSON, Hans
S-113 38 Stockholm (SE)**

(74) Representative: **Sjölander, Henrik et al
Aros Patent AB
P.O. Box 1544
751 45 Uppsala (SE)**

(56) References cited:
**US-A1- 2003 114 898     US-A1- 2004 059 396
US-A1- 2004 116 981     US-A1- 2004 215 272**

**Description**

TECHNICAL FIELD

The present invention generally relates to synchronization of implantable medical devices, and in particular to synchronization of internal clocks of such implantable medical devices with external non-implanted devices.

BACKGROUND

An implantable medical device (IMD), such as a pacemaker, implantable cardioverter or implantable defibrillator, typically has functionality for communicating with an external, non-implanted, device, e.g. programmer, by means of wireless communication. For example, a clinician may use the external device to program the operational parameters of the IMD, e.g. by modifying the pacing mode of the IMD after implantation. The IMDs of today typically also can transmit data to the external device, i.e. supporting bidirectional communication. Such transmitted data could include information of various operational parameters of the IMD and/or physiological and diagnostic data collected by the IMD.

The IMDs presently in the market usually do not store collected physiological and diagnostic real-time data in a buffer before transmission. On the contrary, such physiological data is typically transmitted, in "real time", at the same rate as it is sampled by the IMD. However, the next generation of IMDs will use radio packets and radio frequency (RF) telemetry, where a radio packet contains a relatively large quantity of data samples. As a consequence, sampled data will typically no longer be transmitted on the fly, but first temporarily buffered until a large enough quantity of data is obtained, before transmission as a radio packet to the external device. It is, though, often important for the external device to know the time instance at which the physiological data was sampled. Thus, in order to efficiently process the received physiological data, e.g. by monitoring any significant changes in the data over time, the sampled data is preferably time-stamped.

Time-stamping of physiological data by the IMD is important also for other reasons. Interferences, contact losses, etc. may disrupt the transmission of the radio packets with the data from the IMD to the external device. As a result, the IMD might have to retransmit a particular radio packet a number of times until it is correctly received and decoded by the external device. Without any time-stamping of the data, the external device cannot know the exact time when the sampling actually occurred for the incoming radio packet.

In addition, in the case where the external device receives physiological or diagnostic data both from the IMD and an external diagnostic device, the external device might want to co-process the data from the two independent data sources. For example, the external device could receive intracardic electrogram (IEGM) and/or event marker data from the IMD and surface electrocardiogram (ECG) data from a surface ECG measuring device. If the external device is to co-present the IEGM and surface ECG data on a display screen, the external device has to know the time instance at which the IEGM and ECG data was sampled. This problem is solved if the IMD and preferably also the ECG measuring device time-stamp their respective sampled data before transmission to the external device.

In order to successfully use such a time-stamping, the internal clock of the IMD has to be synchronized with the internal clock of the external device. As a consequence, a reliable and robust synchronization protocol is required for synchronizing the IMD and the external device.

US patent application no. 2003/0114898 discloses a telemetry system enabling RF communications between an IMD and an external device, in which the RF circuitry is normally maintained in a powered down state in order to conserve power. At synchronized wakeup intervals, one of the devices powers up its RF transmitter to request a communications session, and the other device powers up its RF transmitter to listen to the request. The wakeup timers of the two devices are synchronized by one of the devices transmitting a reference time to the other device that adjusts its timer accordingly.

US patent application no. 2004/0215272 teaches a technique for synchronizing the internal clocks of an IMD and an external device, with reduced reliance on periodic polling. The disclosed synchronization technique is based on that one of the devices computes a time drift. This time drift is computed using a calculated drift rate and an elapsed time since a previous synchronization occasion of the clocks. The IMD then polls the external device as a function of this time drift. By applying this technique, the IMD polls the external device at times when the external device is more likely to be receptive to the poll. Once the external device is successfully polled and communication is established, the external device can transmit a reference time to the IMD, which adjusts its internal clocks accordingly.

US patent application no. 2004/0059396 discloses an implantable medical device communication system communicating information between an implantable medical device and at least one slave device by way of a two-wire bus. The implantable medical device includes a communication unit to combine data and power for transmission over the two-wire bus. The transmitted signal is selectively changeable between a first and second voltage. The slave device includes a recovery unit to recover data and power from the received signal.

SUMMARY

In the prior art synchronization protocols, as represented by the above-identified US patent applications, the device transmitting the reference time is not informed whether the receiving device actually received the reference time and adjusted its internal clock accordingly, nor whether the receiving device correctly interpreted the reference time, if received at all. Thus, there is a problem in the prior art synchronization protocols in the lack of robust and reliable feedback and hand shaking in the sense that device initiating the synchronization is not informed whether the other device was actually successfully synchronized or not.

The present invention overcomes these and other drawbacks of the prior art arrangements.

It is a general object of the present invention to provide a robust and reliable synchronization protocol based on data feedback.

It is another object of the invention to provide a synchronization of implantable medical devices (IMDs) with external communicating devices.

It is a particular object of the invention is to provide a synchronization of a clock of an IMD with an external device, allowing usage of the clock for time-stamping physiological data collected by the IMD.

These and other objects are met by the invention as defined by the accompanying patent claims.

Briefly, the present invention involves synchronization of an IMD and an external device. This synchronization is initiated by a master device that is selected from one of the IMD and the external device. The master device reads, in a first time interval, a current first reference time from its internal clock or from an associated time standard clock. This first reference time is associated with the first time interval, in that it represents this first time interval. The master device inserts the first reference time in a synchronization set-up message that is wirelessly transmitted to a slave device being selected from the other of the IMD and the external device. The slave retrieves the first reference time from the message and sets or adjusts its internal clock based on the reference time. The slave further provides a second current reference time and returns it to the master device in an acknowledgement (ACK) response in a second time interval. The slave could provide the second reference time by reading it from its now adjusted internal clock or by calculating it from the first reference time. Thus, the second reference time is provided by the slave based on the first reference time. Upon reception of the ACK response, the master retrieves the included second reference time and investigates whether it is associated with the second time interval. If this is the case, the master device confirms that the slave is successfully synchronized with the master.

This investigating process is preferably implemented by the master device reading a current reference time from its internal or associated clock at the reception of the ACK response. The second reference time is then regarded as associated with the second time interval and the synchronization is successful, provided that the second reference time corresponds to the current reference time, preferably is equal to the current reference time.

This feedback in the synchronization of the invention notifies the master device that the transmitted (first) reference time actually was received by the slave device. In addition, the master device can also confirm, by the comparison of the returned (second) reference time with the reference time read from the master clock, that the slave device set its slave clock to a correct time and within a correct time interval.

If the master device did not receive any ACK response or if the second reference time in the ACK response was not associated with the second time interval, the master preferably reads a new current reference time from its associated master clock and sends a new set-up message to the slave.

Note that if the slave device manages to set its slave clock and return the ACK response within the same time interval as the master transmitted the set-up message, the first and second reference times and the first and second time intervals will be identical. However, it might be possible that the slave is not able to respond directly after reception of the set-up message and the first reference time. In such a case, the second time interval, during which the ACK response is communicated to and received by the master, will be a subsequent time interval. As long as the second reference time provided by the slave is associated with this second time interval, the slave and master are considered synchronized, independent on whether the second time interval is the same as first time interval or a subsequent time interval.

In the case where the slave and master clocks have different clock frequencies so the time intervals used by the master and slave differ, information of the time interval lengths are preferably communicated between the devices and used in the confirming procedure.

The synchronization procedure of the present invention can be extended to involve multiple slave devices, which all then can be synchronized with the master and, thus, with each other. In such a case, the master can transmit (unicast) a set-up message and reference time to each slave device or transmit (multicast or broadcast) a single set-up message and reference time to all slave devices.

In most typical implementations the master is the external device, e.g. in the form of a programmer or data processing system, with the slave as an IMD, or in the case of multiple slaves, the IMD(s) and any external diagnostic device(s).

The synchronization of clocks of the IMD and the external device is important for successful transmission and processing of physiological data collected by the IMD and forwarded to the external device. Such physiological data can temporarily be stored in the IMD before transmission, preferably in the form of radio packets, to the external device. By time-stamping the data or the radio packets using the IMD clock synchronized with the external device, the time instance when the data was sampled is known and can be used by the external device when processing the data. This is in particular true in the case where, e.g. because of high interference levels, a particular radio packet and the data contained therein is delayed due to many re-transmissions. Time-stamping is also important when the external device receives physiological data from multiple sources, e.g. the IMD and an external diagnostic device or such a diagnostic device constituting a part of the external device. In order to successfully co-process the data together, e.g. by displaying the different data together on a screen, the relevant time relationship between the data has to been known. This solved by employing clocks synchronized according to the present invention for time-stamping physiological data in the different devices.

The invention offers the following advantages:

- Provides a robust and reliable synchronization protocol with data feedback;
- A single device can perform the synchronization of all independent clocks in the system;
- Re-synchronization of the involved devices can be performed at any time;
- Enables efficient usage of RF-based transmission of data between IMDs and external devices;
- Provides a possibility of time-stamping physiological data to mark the time of sampling the data;
- Sampled data can be temporarily stored and subsequently transmitted at any suitable time without loss of the relative time relationship of the data;
- Allows discarding physiological data that are no longer relevant; and
- Co-processing and co-displaying physiological data from different devices can be performed using different synchronized clocks.

Other advantages offered by the present invention will be appreciated upon reading of the below description of the embodiments of the invention.

SHORT DESCRIPTION OF THE DRAWINGS

The invention together with further objects and advantages thereof, may best be understood by making reference to the following description taken together with the accompanying drawings, in which:

Fig. 1 is a schematic overview of an embodiment of a synchronization and data processing system according to the present invention;

Fig. 2 is a synchronization signaling diagram according to the prior art;

Fig. 3 is another synchronization signaling diagram according to the prior art;

Fig. 4 is a synchronization signaling diagram according to an embodiment of the present invention;

Fig. 5 is a synchronization signaling diagram illustrating re-initiation of the synchronization in the case of missing reception of synchronization data;

Fig. 6 is a synchronization signaling diagram illustrating re-initiation of the synchronization in the case of failed previous synchronization;

Fig. 7 is a synchronization signaling diagram illustrating acknowledgement requesting in the case of failed previous reception of synchronization data;

Fig. 8 is a synchronization signaling diagram according to another embodiment.of the present invention;

Fig. 9 is a synchronization signaling diagram according to an embodiment of the present invention with different time interval lengths;

Fig. 10 is a synchronization signaling diagram according to an embodiment of the present invention with time delay compensation;

Fig. 11 is a synchronization signaling diagram according to an embodiment of the present invention with multiple slave devices;

Fig. 12 is a synchronization signaling diagram according to another embodiment of the present invention with multiple slave devices;

Fig. 13 is a flow diagram illustrating a synchronization method implementable in a master device according to the present invention;

Fig. 14 is a flow diagram illustrating a synchronization method implementable in a synchronization system according to the present invention;

Fig. 15 is a flow diagram illustrating an embodiment of the sending step of Fig. 13 or 14 in more detail;

Fig. 16 is a flow diagram illustrating an embodiment of the confirming step of Fig. 13 or 14 in more detail;

Fig. 17 is a flow diagram illustrating a synchronization method implementable in a slave device according to the present invention;

Fig. 18 is a schematic block diagram illustrating an embodiment of a master device according to the present invention;

Fig. 19 is a schematic block diagram illustrating an embodiment of a slave device according to the present invention;

Fig. 20 is a schematic block diagram illustrating an embodiment of an implantable medical device according to the present invention; and

Fig. 21 is a schematic block diagram illustrating an embodiment of a data processing device according to the present invention.

## DETAILED DESCRIPTION

Throughout the drawings, the same reference characters will be used for corresponding or similar elements.

The present invention relates to a reliable and robust synchronization of an implantable medical device (IMD), in which an internal clock of the IMD is synchronized with an external communicating device. The synchronized clock can then be used by the IMD for data time-stamping, allowing an efficient processing of such time-stamped data from the IMD in the external device.

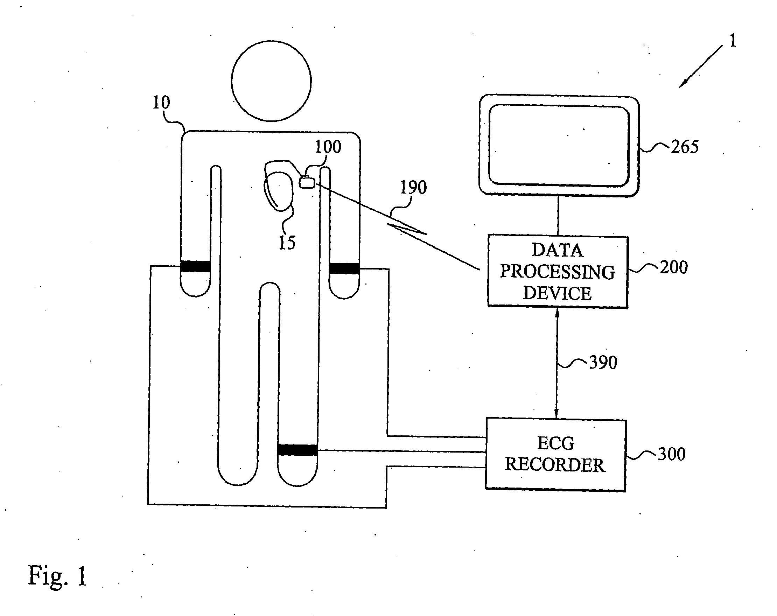

Fig. 1 is an overview of a synchronization and data processing system 1 according to the present invention. The system 1 includes at least one IMD 100 implanted in a patient or subject 10 in need thereof. In Fig. 1, the IMD 100 is illustrated as a device that monitors and/or provides therapy to the heart 15 of the patient 10, such as a pacemaker, defibrillator or cardioverter. However, the present invention is not limited to cardiac-associated IMD but may also be practiced with other implantable medical devices, such as drug pumps, neurological stimulators, physical signal recorders, oxygen sensors, or the like.

The IMD 100 preferably includes functionality for collecting and sampling physiological and diagnostic data, such as intracardic electrogram (IEGM) and/or event marker data in the case of a cardiac-associated IMD. This physiological data and also data representing operational parameters and settings of the IMD 100 might be useful for a clinician and is therefore preferably communicated to an external device 200. As a consequence, the IMD 100 typically includes a communications module to communicate with the external device 200 via a wireless link 190. This wireless transmission could generally be realized using any known non-invasive wireless technique usable in medical applications. A preferred example is radio frequency (RF) telemetry, in which radio packets carrying data are transmitted over the wireless link 190 between the IMD 100 and the external device 200.

The external device 200 of the system 1 is represented by a communicating data processing device in Fig. 1. This data processing device 200 could generally include functionality for monitoring the operation of the IMD 100 based on operational parameters and routine status reports received from the IMD 100. The processing device 200 can also include programmer functionality allowing downloading or programming of the operational parameters of the IMD 100, e.g. by modifying the pacing mode of the IMD 100 after implantation. As was noted in the foregoing, the IMD 100 might also upload physiological data, preferably time-stamped physiological data, to the data processing device 200 over the communications link 190. The processing device 200 could then process the data e.g. in order to find any deviation from the normal patient state. Alternatively, or in addition, the physiological data received from the IMD 100 can be processed by the processing device 200 for presentation on an associated display screen 265.

The data processing device 200 might also be communicating 390 with an external diagnostic devices 300, represented by an electrocardiogram (ECG) recorder in the figure. This external diagnostic device 300 collects physiological and diagnostic data similar to the IMD 100 but operates independently from the IMD 100. Physiological data from the external diagnostic device 300, e.g. surface ECG data, is then forwarded to the data processing device 200 using a communications link 390 between the diagnostic device 300 and the data processing device 200. This communications link 390 could be a wireless link, such as a radio link, or a wired link.

In a particular embodiment of the present invention, this diagnostic device 300 constitutes a part of the data processing device 200. This combined device then includes both communications functionality (communicate with the IMD 100), data recording functionality (record diagnostic data) and data processing functionality (process or co-process data received from the IMD 100 and/or recorded itself).

In the case the data processing device 200 receives physiological data from two independent sources, i.e. the IMD 100 and the ECG recorder 300 in the figure (or the IMD 100 and internal diagnostic device), the processing device 200 could be configured for co-processing this data. For example, IEGM data from the IMD 100 and surface ECG data from the ECG recorder 300 could be co-presented together on the display screen 265. In such a case, it is important that the time instance at which the respective data was sampled or collected is known. This problem is solved according to the present invention by the IMD 100 and preferably the external diagnostic device 300 time-stamping their respective collected physiological data.

In order to effectively provide a meaningful data time-stamping, the internal clock of the IMD 100 has to be synchronized with the data processing device 200. The synchronization can be initiated by either of the IMD 100 and the data processing device 200 e.g. before or during setting-up a communications session on the communications link 190, or some other time during the communications session.

Furthermore, due to a variety of factors, the internal clocks in the IMD 100 and the data processing device 200 may run at different rates. Accordingly, one internal clock drifts with respect to the other over time, and the internal clocks lose synchronicity. As a consequence, the IMD 100 and the data processing device 200 is preferably also re-synchronized e.g. at each session set-up, at some predefined time instances or periodically.

The synchronization procedure employed between the IMD 100 and the data processing device 200 may likewise be employed between the external diagnostic device 300 (ECG recorder) and the data processing device 200 or between the external diagnostic device 300 and the IMD 100, if these two devices can communicate with each other. Correspondingly, in situations where the system 1 includes more than one IMD 100, all of these IMDs 100, or at least those IMDs that collect physiological data, are preferably synchronized with the data processing device 200.

According to the present invention, the expression "master device" represents the communicating device in the synchronization system 1 that initiates the synchronization procedure by transmitting a reference time according to the invention. This master device could be the IMD 100 or an external device 200, 300, preferably the data processing device 200. Correspondingly, a "slave device" denotes, according to the present invention, the other device communicating with and to be synchronized with the master device. The slave device is then preferably the other of the IMD 100 or the external device 200, 300. Note that a master device, e.g. the data processing device 200, can synchronize multiple, i.e. at least two, slave devices, e.g. the IMD 100 and the ECG recorder 300.

The label "master device", thus, denotes the initiating device, which can be different from one synchronization procedure to the next. For example, in a first synchronization procedure, the IMD 100 transmits a reference time over the wireless link 190 to the data processing device 200 for the purpose of initiating the synchronization procedure. Thus, the IMD 100 is the master device with the processing device 200 as slave in this first procedure. In a subsequent re-synchronization procedure, the processing device 200 could be the master and the slave is then the IMD 100.

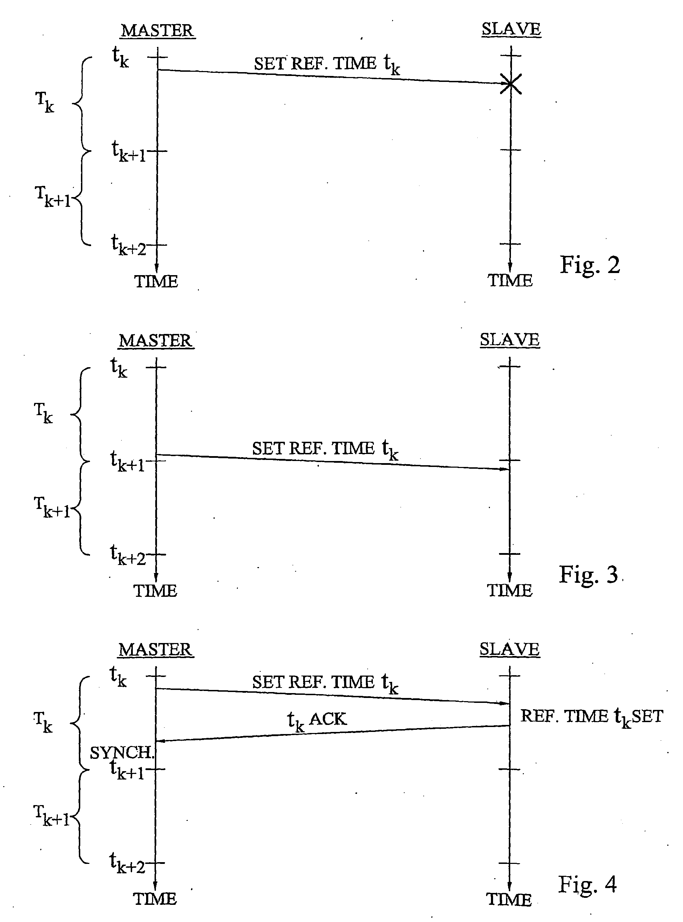

Before describing the synchronization procedure according to the present invention, a discussion of the problems with the prior art synchronization protocols without any reliable feedback and confirming procedures first follows in connection with Figs. 2 and 3.

Fig. 2 illustrates a signaling diagram illustrating synchronization traffic between a master and a slave device according to the prior art. The master has an internal clock or at least access to a clock, if not provided as internal clock. The master retrieves, from this clock, a current reference time $t_k$ that should be communicated to the slave. The slave should then adjust its internal clock accordingly. However, the transmission of the reference time $t_k$ might be unsuccessful e.g. due to a (temporary) high interference. As a consequence of this high interference (or some other transmission disturbing source), the slave does not successfully receive the reference time. If the slave does not inform the master of the unsuccessful reception, the master has no knowledge of the failed synchronization. Thus, the master and slave will run asynchronous following this failed synchronization according to the prior art techniques.

Fig. 3 illustrates another possible synchronization procedure according to the prior art. As is well known in the art, a clock has an intrinsic (fixed or adjustable) clock frequency, represented in the Fig. 3 by time intervals $T_k$, $T_{k+1}$

(clock frequency = $\dfrac{1}{\text{length}(T_k)}$). Each such time interval $T_k$, $T_{k+1}$ is then associated with a given reference time $t_k$, $t_{k+1}$. For example, assume that the clock is implemented as a wrap-around counter counting up from 0 to K-1 and wrapping from K-1 to 0. The time period between the time instance where the clock counts from k-1 to k to the instance where the clock counts from k to k+1 represents such a time interval. The association between a time interval $T_k$, $T_{k+1}$ and a reference time $t_k$, $t_{k+1}$ means that during the time interval $T_k$ ($T_{k+1}$) the clock is $t_k$ ($t_{k+1}$).

If the master device transmits the reference time $t_k$ rather late in the time interval $T_k$, it might be possible that the reference time $t_k$ is not received by the slave until in the next time interval $T_{k+1}$. The slave device, thus, adjusts its clock by setting it according to the reference time $t_k$, whereas the clock of the master device now is $t_{k+1}$. The two clocks will then run asynchronous.

Note, though, that a simple reception acknowledgement will not solve the problem presented in Fig. 3, since the master device will then, in response to the acknowledgement, erroneously think that the slave, has correctly adjusted its clock and that the clocks of the two devices run synchronous.

The present invention solves the synchronization problems discussed above in connection with Figs. 2 and 3 by providing a reliable and robust synchronization protocol, an embodiment of which is illustrated in the signaling diagram of Fig. 4.

The synchronization procedure is initiated by the master that retrieves or reads a current reference time $t_k$ from its internal or associated block. This reference time $t_k$ is associated with the current time interval $T_k$. The length of the time interval $T_k$ is preferably relatively long compared to the time it takes to communicate with the slave, in order to potentially allow both transmission of a synchronization set-up message and reception of an acknowledgement (ACK) response within a same time interval.

Thus, the reference time $t_k$ is wirelessly transmitted from the master device in the form of a synchronization set-up message to the slave device. Upon reception of this message, the slave sets or adjusts its internal or associated clock accordingly. The slave then composes an ACK response to be transmitted to the master device. This ACK response comprises the reference time $t_k$ currently displayed or represented by the slave clock. Since the slave clock has been adjusted based on the reference time $t_k$ received from the master and the reference time $t_k$ in the ACK response is retrieved from the slave clock after the adjustment, the reference time $t_k$ in the ACK response is based on the reference time $t_k$ from the master device. In an alternative implementation, in particular in the case where the slave adjusts its clock and responds rather quickly so that the reference time $t_k$ in the ACK response will represent the same time as the reference time $t_k$ in the received synchronization message, the slave can provide the reference time $t_k$ to be included in the ACK response directly from the received reference time $t_k$, without first adjusting the clock and then retrieving the reference time $t_k$ therefrom.

In either case, once the master device receives the ACK response it confirms that the slave is synchronized with the master provided that the reference time $t_k$ included in the ACK response is associated with the time interval $T_k$, during which the master received the response. As is represented by the embodiment illustrated in the Fig. 4, transmission of the reference time $t_k$ from the master to the slave, the setting of the slave clock and the return of the ACK reference time $t_k$ can all take place within a single time interval $T_k$. Since the received ACK reference time $t_k$ is associated with the current time interval $T_k$, the master confirms that the slave has been successfully synchronized.

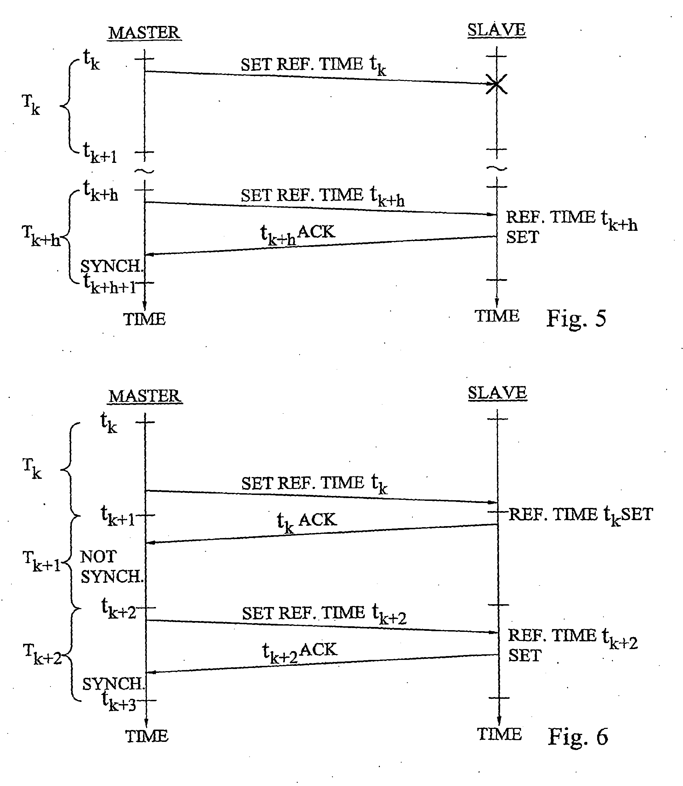

Fig. 5 illustrates the signal diagram in the case of failed transmission of the synchronization set-up message and the initial reference time $t_k$. In this figure, some external source might interfere with the transmission or the transmission fails due to some other cause. As a consequence, the slave did not successfully receive the reference time $t_k$. In such a case, the slave could return a negative acknowledgement (NACK) response to the master, urging the master to send a new set-up message and reference time $t_k$. In an alternative implementation, and in particular in cases where the slave did not receive any set-up message at all, the master device could be configured for re-initiating the synchronization procedure if no ACK or NACK response has been received from the slave within a predefined period of time from transmission of the first set-up message. The master device then composes a new set-up message and includes the reference time $t_{k+h}$ associated with the current time interval $T_{k+h}$. The message is transmitted to the slave, which sets its clock using the received time reference $t_{k+h}$. An ACK response is composed with a return time reference $t_{k+h}$ provided based on the received time reference $t_{k+h}$. If the master receives this ACK response within the time interval $T_{k+h}$, the master and slave are confirmed synchronized.

Fig. 6 illustrates another signal diagram according to the present invention illustrating late transmission of the set-up message and reference time $t_k$. In this example, the slave device received the set-up message first at the near end of the current time interval $T_k$. As a consequence, the slave did not have time to set its clock until the next time interval $T_{k+1}$. Thus, the slave clock is set to $t_k$, whereas the master clock is now $t_{k+1}$. The slave device composes the ACK response and inserts a current reference time $t_k$ as read from its associated clock or calculated from the received

reference time $t_k$ and sends it to the master. The master device investigates the reference time $t_k$ in the ACK response in order to elucidate whether it is associated with the current time interval $T_{k+1}$, during which the ACK response was received. However, the received reference time $t_k$ is associated with the preceding ($T_k$) and not the current $T_{k+1}$ time interval and the slave is accordingly not regarded as successfully synchronized with the master.

In the sequence above, the ACK response from the slave will not be accepted by the master because the response is received too late, i.e. in the "wrong" time interval. The master cannot be certain in what time interval the slave set its clock, was it in the preceding time interval $T_k$ or in the current interval $T_{k+1}$? The only safe way is to re-run the synchronization set-up. As a consequence, the master provides a second set-up message and a second reference time $t_{k+2}$ associated with the current time interval $T_{k+2}$. The message is transmitted to the slave that sets its clock according to the included reference time $t_{k+2}$. The slave also composes a second ACK response with the reference time $t_{k+2}$ provided from the set slave clock or from the received reference time $t_{k+2}$ and returns it to the master. The master confirms that the slave and master are now synchronized since the received reference time $t_{k+2}$ is associated with the current reference time $T_{k+2}$.

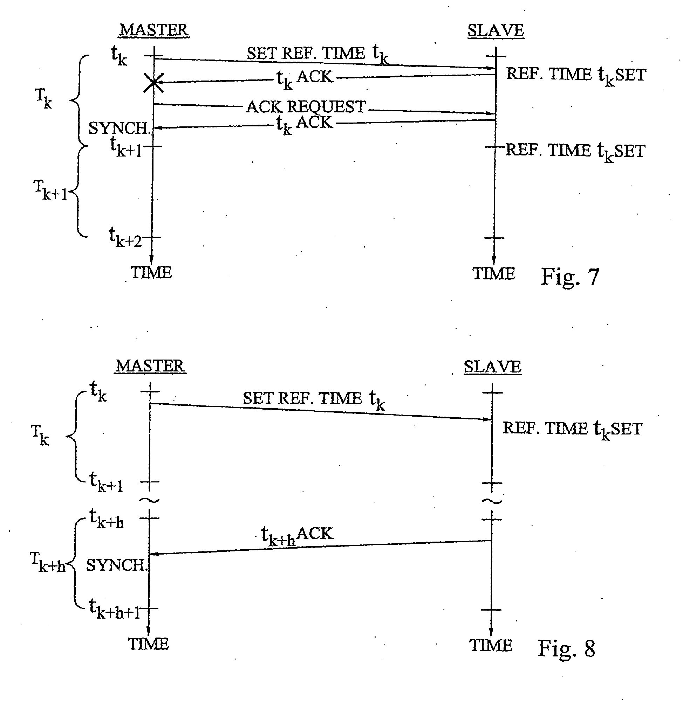

As is illustrated in the signaling diagram of Fig. 7, the communications link between the master and slave may be interfered so that the ACK response from the slave fails to reach the master. The master device then preferably returns an ACK request message, urging the slave to respond once again. The slave then re-transmits the previous ACK response or composes a new ACK response with a possible new included reference time $t_k$. In either case, the ACK response is once more transmitted to the master device. If the new ACK response is received within the time interval $T_k$, the master knows that the two devices are synchronized. In the case the ACK response is delayed, the synchronization procedure might have to be re-run.

Note that the slave does not necessarily have to return the ACK response within the same time interval as the set-up message and reference time was received from the master device. This is schematically illustrated in Fig. 8. In this figure, the slave device received the set-up message within the time interval $T_k$. The slave sets its associated clock according to the reference time $t_k$ included in the received set-up message. However, the slave might not be able to directly respond to the set-up message. For example, the slave can temporarily be busy with other tasks, for example collecting physiological data. This means that the slave will return the ACK response not until some later time interval $T_{k+h}$. In such a case, the ACK response then includes the current reference time $t_{k+h}$ as retrieved from the slave clock. If the master device successfully received this ACK response within the time interval $T_{k+h}$, the two devices are regarded as synchronized.

The time intervals according to the present invention can represent the smallest time increment necessary for the sampling system with respect to the frequency content of the physiological signal. However, also longer time intervals than this smallest time increment could be employed. For example, assume a sampling or clock rate of 250 Hz. This means that the smallest time step is $\dfrac{1}{250}$ = 4 ms. A time interval could then be 4 ms long. In another embodiment, a longer time interval is used for synchronization purposes. A wrap-around counter clock having a clock frequency of 250 Hz and starting from the clock state 0, will four ms later be 1, is 2 eight ms later and so on. It could then be possible that a time period of 4 ms is too short for allowing the master and slave sufficient time to complete a synchronization procedure. In such a case, the time interval used for synchronization purposes could be longer than this smallest 4 ms time step, e.g. being 40 ms. This means that the reference times $t_k$, $t_{k+1}$, $t_{k+2}$, ... could then represent 0, 10, 20, ...

If one of the master and slave employs a time interval longer than the smallest time step and/or if the clocks of the two devices run at different clock frequencies, information of the employed time interval length is preferably communicated before initiating the synchronization procedure or during the procedure.

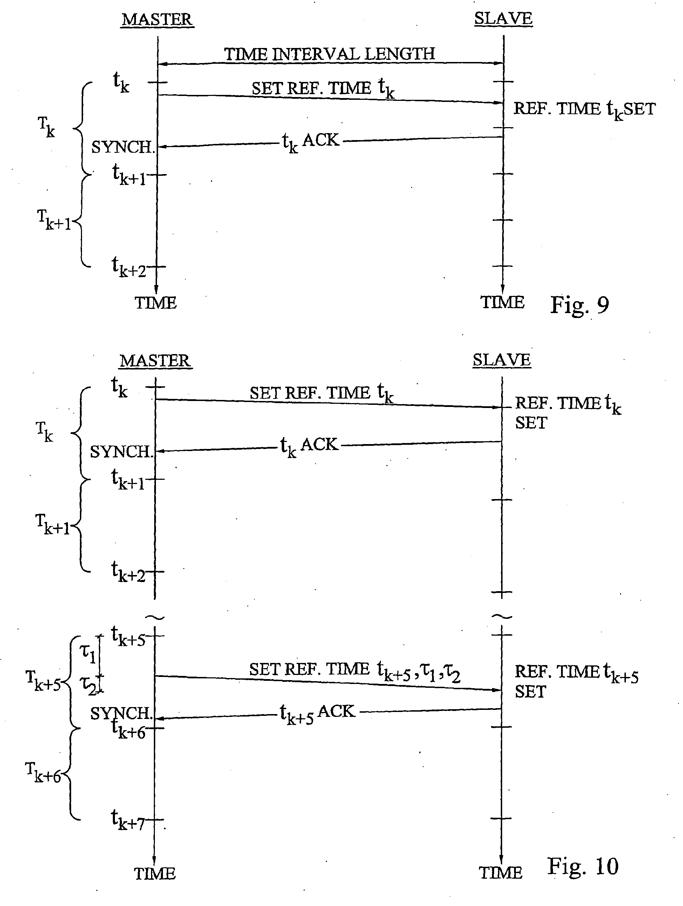

Fig. 9 illustrates a signal diagram of the synchronization procedure of the present invention in the case where the clock frequency of the slave clock is twice the clock frequency of the master clock. This could be seen as an illustrative example of the case where the time length of the slave is p times the corresponding time length of the master, where p is a positive number.

In the figure, time interval length information is communicated between the master and the slave prior or during the synchronization procedure. This information could include the time interval length employed by one of the devices and/or the clock frequency of that device. Note further, that it may be enough if one of the master and slave communicates its time interval length information to the other of the master and slave. For example, the master may transmit information of its associated interval length to the slave included in the set-up message together with the reference time $t_k$ or before transmission of the set-up message. The slave sets its clock as previously and composes the ACK response. When composing this ACK response, the slave clock has increased by one time unit, implying that the ACK response is composed in a slave time interval following the time interval, during which the set-up message was received and the slave clock was set. However, since the master has communicated its length information to the slave, the slave knows that the master device clock has not increased with one time unit Thus, the slave device preferably compensates for this time length difference when providing the reference time $t_k$ to be included in the ACK response.

In an alternative implementation, the slave device communicates its slave time length or slave clock frequency to the master device. The master then compensates, upon reception of the ACK response, for the different time interval lengths.

In the previous figures, the time intervals of the master and slave device, though possible different, has been illustrated as fixed. The present invention is, however, not limited thereto. For example, the clock of one of the devices might have an adjustable clock frequency. As a result, the length of the time interval associated with this device might change during operation. In such a case, this device preferably informs the other device about the new interval length or compensates itself for the new length in a following (re-)synchronization procedure.

Fig. 10 illustrates another signaling diagram according to the present invention. As is illustrated in the upper portion of the figure, the slave can set its internal clock once it received the time reference $t_k$ from the master. Depending on the particular slave clock and the implementation of the clock setting functionality, the present clock indication could be set to $t_k$ without affecting the clocking times, i.e. the time instance at which the clock is increased by one time unit. In such a case, the master and slave clock will still run syntonous, i.e. the master and slave clocks are increased by one time unit at the same time instances.

In an alternative implementation, a new time interval can start running from the time instance at which the slave clock was set based on the received reference time $t_k$. As a result, which is illustrated in the upper portion of the figure, the slave clock will lag behind the master clock with a period of time that corresponds to the period between the start of the current time interval $t_k$ as defined by the master clock until the instance when the slave set its clock. This is generally no problem for the synchronization system, since such a small time lag can often be neglected. However, if such a delay or lag is relatively constant or if it can be estimated, the master and slave can compensate for this time offset of their clocks.

As is illustrated in the lower portion of Fig. 10, assume that the time interval $T_{k+5}$ used for synchronization purposes does not represent the smallest time increment representable by the master clock. In such a case, the time period $\tau_1$ elapsed from start of the current time interval $T_{k+5}$ to the transmission of the set-up massage can be communicated to the slave device together with the current reference time $t_{k+5}$. In addition, if the master can estimate the time period $\tau_2$ which the set-up message transmission will take, also that estimated time period $\tau_2$ can be communicated to the slave device. In an alternative embodiment, only the time period $\tau_1$ is forwarded to the slave, which then estimates the transmission associated period $\tau_2$. In this case, the slave device sets its clock based on both the received reference time $t_{k+5}$ and the time periods $\tau_1$, $\tau_2$. Compared to the upper portion of Fig. 10, the slave can, in the lower portion of Fig. 10, compensate for any known or estimated delays so that both synchronization and synotonization of the master and slave clocks are obtained.

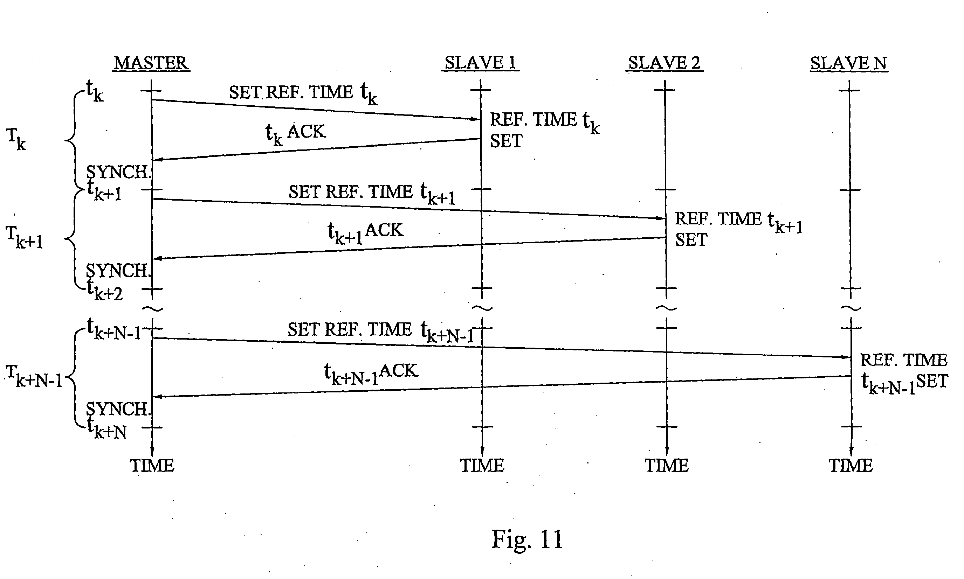

In the discussion of the present invention above in connection with Figs. 4 to 10, a single slave device has been synchronized with a master device. The synchronization provided according to the invention can also be applied for synchronizing multiple slaves with a single master as is schematically illustrated in Fig. 11. In this figure N slave devices, where N is an integer larger than one, is synchronized with the master. This synchronization can be performed basically according to N separate point-to-point synchronizations. Thus, in a first time interval $T_k$ the master sends a set-up message with a first reference time $t_k$ associated with the first time interval $T_k$ to a first slave. This slave sets its clock using the first reference time $t_k$ and returns an ACK response message with a time reference $t_k$ provided based on the first reference time $t_k$. If the time reference $t_k$ in the ACK response is successfully received by the master during the time interval $T_k$, with which the reference $t_k$ is associated the first slave is confirmed synchronized. Correspondingly in a second time interval $T_{k+1}$ the master transmits, to a second slave, a second set-up message including a second reference time $t_{k+1}$ associated with the second interval $T_{k+1}$. This second slave sets its internal clock accordingly and returns an ACK response with a reference time $t_{k+1}$. The master then checks if this reference time $t_{k+1}$ is associated with the current time interval $T_{k+1}$ and if so confirms that the second slave is correctly synchronized. This procedure is repeated for all N slaves as schematically illustrated by the figure.

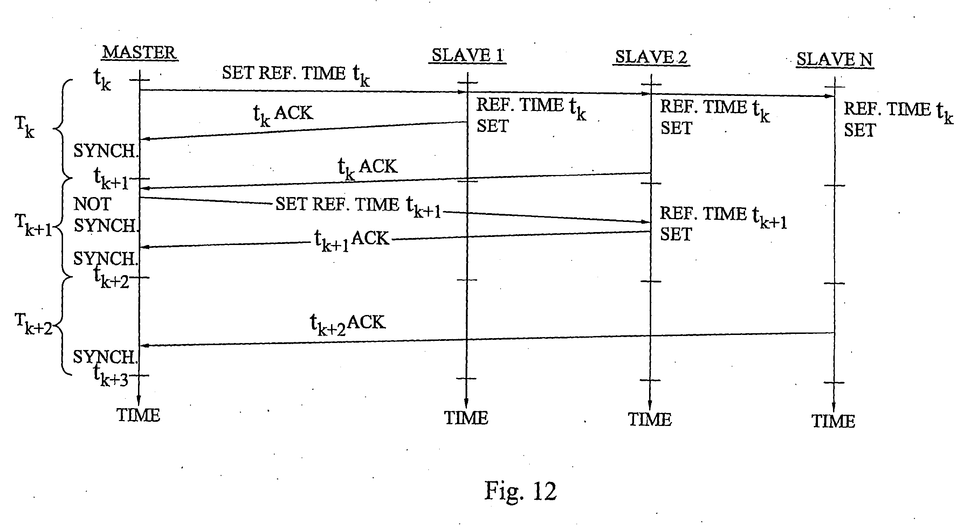

Instead of basically running N separate synchronization procedures for the purpose of synchronizing N slaves with the master, a combined synchronization can be performed as is illustrated in Fig. 12. In this figure, the master multicasts or broadcasts a single synchronization set-up message with reference time $t_k$ to the N slaves to be synchronized. The different slaves then adjust their respective clocks based on the reference time $t_k$ in the set-up message. Different process steps can then follow for the different slaves.

For example, a first slave responds with a correct ACK within the same time interval and is regarded as successfully synchronized. A second slave cannot respond directly after reception of the set-up message, e.g. because the slave is occupied with some other task. As a consequence, the ACK response is transmitted rather late within the time interval $T_k$ and does not reach the master until the next time interval $T_{k+1}$. However, since the reference time $t_k$ included in the ACK response is associated with the time interval $T_k$ and not the reception interval $T_{k+1}$, the slave cannot be regarded as synchronized. The master can then re-run a separate synchronization with this particular slave by unicasting a new set-up massage to the slave. Correspondingly, the $N^{th}$ slave cannot respond until the time interval $T_{k+2}$. However, this slave includes the reference time $t_{k+2}$ associated with this interval $T_{k+2}$ in its ACK response. The master can then grant successful synchronization of the slave.

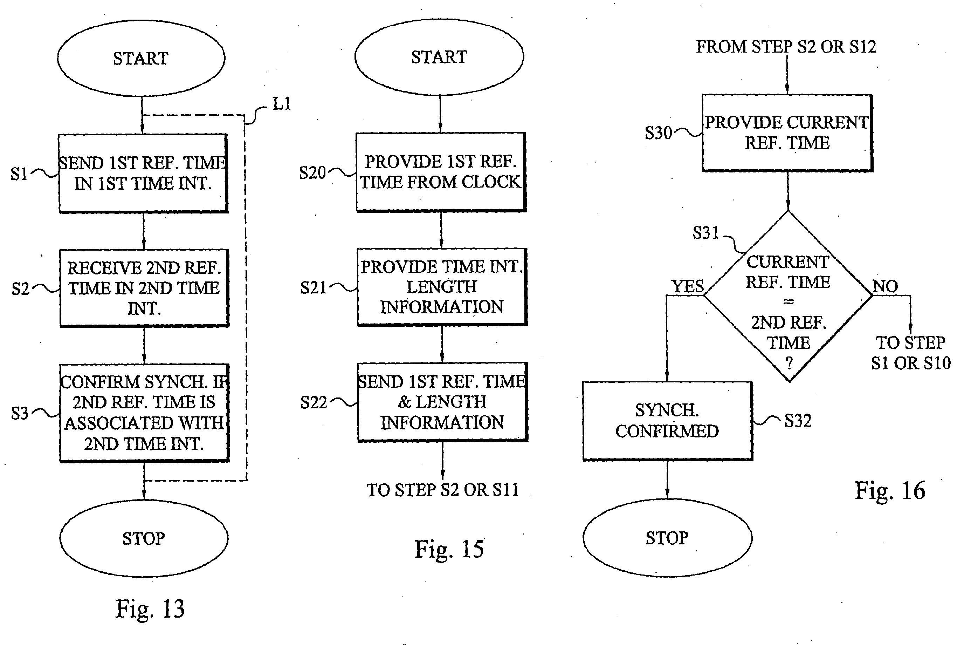

Fig. 13 is a flow diagram illustrating the operation of a master device in a synchronization method according to the present invention. The method starts in step S1, where the master, being one of an IMD and an external device, sends, in a first time interval, a first reference time associated with the first interval to a slave, being the other one of the IMD and external device. In a second step S2, the master receives a second reference time from the slave in a second time interval. This second reference time is provided by the slave based on the previously transmitted first reference time. In a next step S3, the master confirms that the slave is synchronized with the master if the received second reference time is associated with the second time interval.

The master preferably re-initiates the synchronization procedure, which is schematically illustrated by the line L1, if the synchronized is not confirmed due to that the second reference time is not associated with the second time interval.

The method then ends.

The master device can generally perform this synchronization at any time. The synchronization could, for example, be a part of the set-up procedure for a communications session between the master and the slave. Alternatively, or in addition, the synchronization can be performed during such a communications session. In a further alternative, the master could be configured for performing the synchronization at predefined time instances, e.g. periodically.

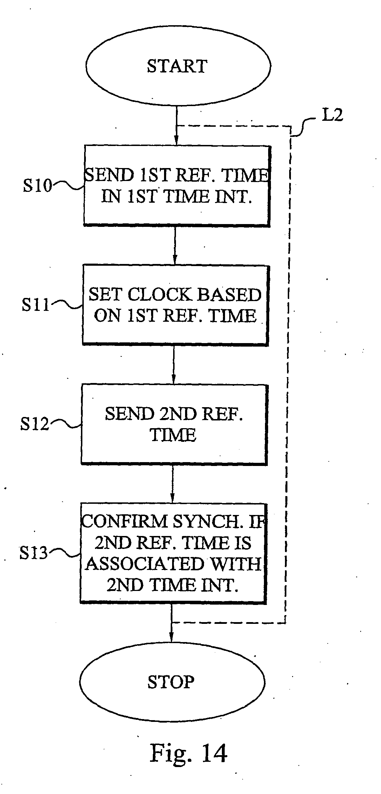

Fig. 14 is a flow diagram illustrating the operation of the master device and the slave device in a synchronization method according to the present invention. The method starts in step S10, where the master transmits the first reference time to the slave. This step corresponds to step S1 in Fig. 13 and is not further described. In a next step S11, the slave sets it internal or associated clock based on the received first reference time. The slave then provides a second reference time based on the received first reference time. This second reference time can be read from the slave clock set using the first reference time or be generated directly using the first reference time. The slave sends the second reference time to the master in step S 12 in a second time interval. Upon reception of the second reference time, the master investigates whether the slave can be confirmed synchronized in step S 13, i.e. whether the second time reference is associated with the second time interval. This step S 13 corresponds to step S3 in Fig. 13 and is not further discussed.

In the case of failed synchronization or in the case of a subsequent re-synchronization, which is schematically illustrated by the line L2, the synchronization protocol of the present invention is performed once more starting from step S10. The method then ends following a confirmed synchronization in step S 13.

Fig. 15 is a flow diagram illustrating an embodiment of the sending step S1 of Fig. 13 or S10 of Fig. 14. The method starts in step S20, where the master device provides the first reference time from its internal or associated clock. This providing step S20 could be realized by the master device reading the first reference time from its associated master clock. In a next optional step S21, the master device provides information of the length of the time interval it currently uses for synchronization purposes. This interval length could be 1/clock frequency of the master clock. However, in alternative implementations other (longer) interval lengths could be employed in the synchronization compared to other operations, including data sampling, in which the clock frequency is used. The interval length information and the first reference time are then sent to the slave in step S22. These two data items could be co-transmitted in a single synchronization set-up message or transmitted separately. The method then continues to step S2 of Fig. 13 or S11 of Fig. 14.

Fig. 16 is a flow diagram illustrating an embodiment of the confirming step S3 of Fig. 13 or S13 of Fig. 14 in more detail. The method continues from step S2 of Fig. 13 or S12 of Fig. 14. In a next step S30, the master devices provides a current reference time from its associated master clock. Thus, the master device investigates its clock and retrieves the reference time of the clock representing the current time at the reception of the second reference time from the slave. In a next step S31, the master compares the current reference time from its master clock with the second reference time from the slave. If the two reference times correspond to each other, the received second reference time is associated with the second time interval in which it was received. The synchronization is then successfully confirmed in step S32 and the method ends. If the two time references do not correspond to each other as determined in step 531, the synchronization should be re-run and the method continues to step S1 of Fig. 13 or S10 of Fig. 14.

In the case of master and slave clocks implemented as wrap-around counters so that the reference times will be zero or a positive integer, the second reference time is regarded as corresponding to the current reference time in step S31 if the two reference times are equal. In other clock implementation, it might be more suitable to regard the second reference time as corresponding to the current reference time in step S31 if the second reference time fulfills the following requirement:

$$\text{current reference time} - \alpha < \text{second reference time} < \text{current reference time} + \alpha$$

Thus, the second reference time then corresponds to the current reference time even if not completely equal thereto as long as it is within a predefined time distance $\alpha$ from the second reference time.

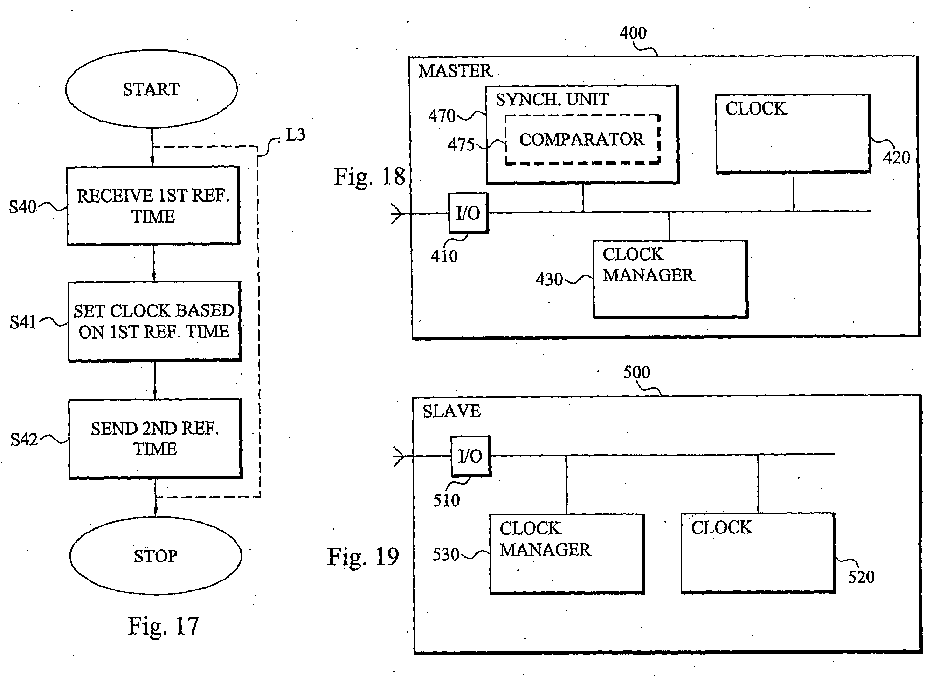

Fig. 17 is a flow diagram illustrating the operation of a slave device in a synchronization method according to the present invention. The method starts in step S40, where the slave, being one of an IMD and an external device, receives, in a first time interval, a first reference time associated with the first interval from a master, being the other one of the IMD and external device. In a second step S41, the slave sets or adjusts its associated slave clock based on the received first reference time. The slave then provides a second reference time based on the first reference time. This second reference time can be retrieved from the slave clock set using the first reference time or be generated directly using the first reference time. The slave then sends the second reference time to the master in step S42 in a second time interval. This step S42 basically corresponds to step S12 in Fig. 14 and is not further discussed. If the second reference time is successfully received by master and the reference time is associated with the second time interval, the two devices are synchronized and the method ends. If not successfully synchronized, the slave preferably receives a new reference time from the master, which is schematically illustrated by the line L3.

The methods according to the present invention may be implemented as software, hardware, or a combination thereof. A computer program product implementing the methods or a part thereof comprises software or a computer program run on a general purpose or specially adapted computer, processor or microprocessor. The software includes computer program code elements or software code portions that make the computer perform the methods using at least one of the steps previously described in Figs. 13, 14 or 17 and preferably also in Figs. 15 and 16. The program may be stored in whole or part, on, or in, one or more suitable computer readable media or data storage means such as a magnetic disk, CD-ROM or DVD disk, hard disk, magneto-optical memory storage means, in RAM or volatile memory, in ROM or flash memory, as firmware, or on a data server.

Fig. 18 is a schematic block diagram of a master device 400 according to the present invention. The master device 400 includes a general input and output (I/O) unit 410 adapted for wireless and optionally wired communication with other devices, i.e. operating as a receiver and transmitter (or transceiver). The I/O unit 410 is in particular configured for transmitting synchronization set-up messages with a reference time to slave devices to be synchronized with the master 400. The I/O unit 410 further receives ACK responses from the slaves.

A master clock 420 is provided in the master device 400 or alternatively arranged outside of the master device 400 but accessible for the master 400. This clock 420 is used by the master 400 for providing reference times and preferably also for investigating whether to confirm a slave to be synchronized with the master clock 420. Any clock implementation 420 that can represent time can be used according to the present invention. A typical implementation of the clock 420 could be a counter and preferably a wrap-around counter. The clock 420 has an intrinsic clock frequency or rate that defines a minimum time period representable by the clock 420. This minimum time period can be used by the master device 400 as a time interval in the synchronization operation according to the present invention. Alternatively, a longer time period than this minimum period is used as synchronization associated time interval.

The master 400 also includes a clock manager 430 for managing the operation of the master clock 420. The manager 430 in particular retrieves reference times from the clock 420 that will be transmitted by the I/O unit 410 to a slave device. The manager 430 preferably also retrieves a reference time from the clock 420 when the I/O unit 410 receives an ACK response from a slave. The clock manager 430 can also be employed for setting and re-setting the clock 420 based e.g. upon a received clock set command from an external unit. This external unit could be a clock reference unit that provides GPS (Global Positioning System) derived time UTC (Universal Time) time or GMT (Greenwich Mean Time) time.

A synchronization unit 470 is provided in the master device 400 and is used for investigating received ACK responses from the slaves to be synchronized with the master 400. This synchronization unit 470 checks whether the time reference in a received ACK response is associated with the time interval during which the I/O unit 410 received the ACK response. In the case that the master 400 and the slave use different time interval length, the I/O unit 410 can have received a notification of the time interval length of the slave. In such a case, the synchronization unit 470 preferably uses this information in the synchronization procedure for compensating between any interval length differences, unless already performed by the slave.

The synchronization unit 470 preferably includes a time reference comparator or comparing unit 475, or such comparing functionality could alternatively be implemented directly in the synchronization unit 470. When the I/O unit 410 receives an ACK response from a slave, the synchronization unit 470 or some other unit in the master 400 retrieves the time reference included in the ACK response and forwards it to the comparator 475. At the ACK reception, the clock manager 430 retrieves a current reference time by investigating and reading the master clock 420. This current reference time is forwarded to the comparator 475. The comparator 475 then compares the two reference times and if they correspond to each other, e.g. being equal or the absolute difference between the time references is smaller than a predefined threshold, the received time reference is regarded as associated with the time interval during which the I/O unit 410 received the time reference. As a consequence, the synchronization unit 470 and the master device 400 then confirm that the slave is synchronized with the master 400.

Once the synchronization unit 470 has confirmed successful synchronization, it may optionally inform the slave using the I/O unit 410 that the slave is now successfully synchronized.

If the synchronization is unsuccessful so that the received reference time does not correspond to the current reference time, the synchronization unit 470 could urge the clock manager 430 to retrieve a new reference time from the clock 420 which will then be transmitted by the I/O unit 410 in the form of a new synchronization set-up message.

Furthermore, if the I/O unit 410 did not successfully receive (demodulate and decode) the ACK response, it preferably returns an ACK request message to the transmitting slave device.

The units 410 to 475 of the master device 400 may be provided as hardware, software or a combination of hardware and software. The master device 400 can be an IMD or an external device communicating with an IMD, e.g. a programmer or data processing device.

Fig. 19 is a corresponding schematic block diagram of a slave device 500 according to the present invention. The slave device 500 includes an I/O unit 510 adapted for wireless and optionally wired communication with other devices. The I/O unit 510 is in particular configured for receiving synchronization set-up messages with included reference time from a master device. The slave 500 further uses the I/O unit 510 for returning ACK responses with included reference time to the master device.

The slave 500 also includes a slave clock 520 to be synchronized with the master device. Correspondingly to the master clock, any clock implementation that can represent time can be used for the slave clock 520, with a wrap-around counter as a preferred example. Note that the clock frequency of the slave clock 520 does not necessarily have to be the same as for the master clock. In such a case, time interval length information is preferably communicated between the slave 500 and the master 400.

A clock manager 530 is implemented in the slave 500 for managing the operation of the slave clock 520. The manager 530 in particular sets or adjusts the clock 520 based on the reference time received by the I/O unit 510 from the master device. The clock manager 530 furthermore provides a return reference time based on the received reference time. This return reference time could be read from the adjusted clock 520 or calculated directly from the received reference time. In either case, this return reference time is provided to the I/O unit 510 that forwards it to the master device for use in confirming the synchronization.

The units 510 to 530 of the slave device 500 may be provided as hardware, software or a combination of hardware and software. The slave device 500 can be an IMD or an external device communicating with an IMD, e.g. a programmer or data processing device.

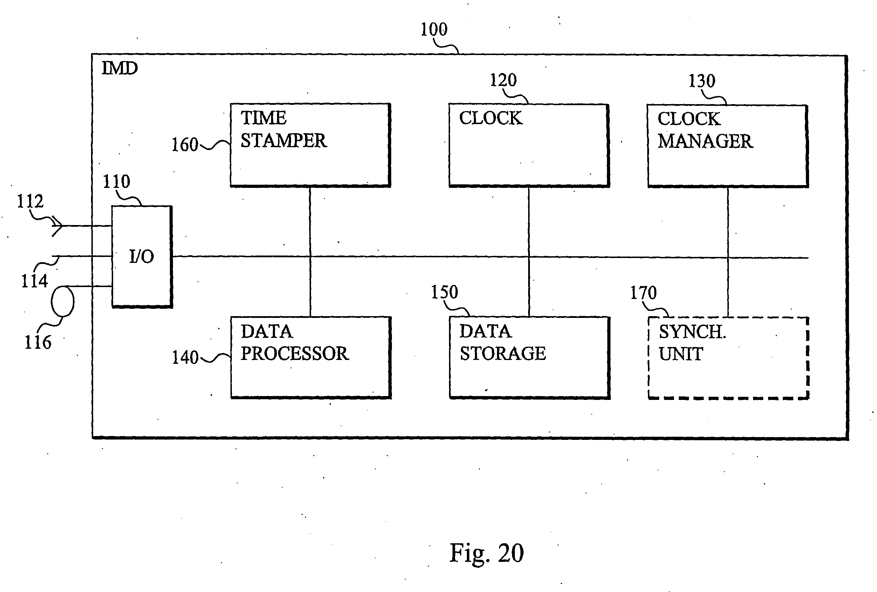

Fig. 20 is a schematic block diagram of an IMD 100 according to the present invention. This IMD 100 could generally be any IMD that has a clock 120 that should be synchronized with a corresponding clock of an external device and that can communicate with the external device by means of wireless transmissions. Typical non-limiting examples of an IMD 100 according to the invention include a pacemaker, implantable defibrillator or implantable cardioverter. Further examples include a drug pump, a neurological stimulator, a physical signal recorder, an oxygen sensor, or the like.

The IMD 100 includes an I/O unit 110 for communication with the external device. This I/O unit 110 includes functionalities for processing incoming and outgoing data messages, including modulator/demodulator and coder/decoder functionality. The I/O unit 110 is further preferably connected to an antenna arrangement 112 used for transmitting and receiving radio packets to and from the external unit, respectively. However, the I/O unit 110 could also or alternatively use other forms of wireless techniques than radio packets when communicating with the external device. The I/O unit 110 could for example use an inductive antenna 116 for external wireless communication.

In a preferred embodiment of the invention, the I/O unit 110 is also connected via a lead or wire 114 to a sensor or probe used for collecting physiological data and measuring physiological parameters in the body of the patient in which the IMD 100 is implemented. This physiological data can e.g. be IEGM, ECG, electromyography (EMG), electroencephalography (EEG), electrooculography (EOG), event marker, respiration, blood pressure and/or oximetry related data. This physiological data is collected and sampled by a data processor 140 of the IMD 100 using the I/O unit 110 and the lead 114 with sensor. The physiological data is then preferably time-stamped using a time stamper or stamping unit 160. This time stamper 160 receives the required time data from a slave clock 120 implemented in the IMD 100. By time-stamping the data collected by the data processor 140, the relevant time instance at which the physiological data was sampled can later be determined by the IMD 100 itself or an external unit to which the time-stamped data subsequently is transmitted.

Once the data has been time stamped by the time stamper 160 it can be directly transmitted to an external unit using the I/O unit 110 and the RF antenna 112 or inductive antenna 116. In an alternative implementation, the data is first temporarily stored in a data storage or buffer 150 provided in the IMD 100. This is in particular advantageous in the case where the data is transmitted in radio packets. In such a case, physiological data can be stored in the storage 150 as it is collected by the data processor 140 (and optionally time stamped by the time stamper 160) until enough data is obtained to compose a radio packet. It could also be advantageous for other reasons to store the data before transmission to the external device. For example, data transmission could be performed only at certain predefined time instances when the external device is likely to be active. Alternatively, the data is only transmitted to the external device upon a request therefrom.

Instead, or alternatively, to time-stamping the physiological data, radio packets containing the data or some other block of multiple data samples could be time stamped by the time stamper 160. This means that data is not time-stamped per data sample but per radio packet or data block.

The clock 120 of the IMD 100 that is used for time-stamping purposes is synchronized with the external device using the synchronization protocol of the invention. The IMD 100 can include a single clock 120 as is illustrated in the figure, which then will be used both for data sampling and other internal operations requiring clocking. However, the IMD 100 could alternatively include multiple clocks 120 used for different purposes, e.g. one dedicated synchronization clock used in the synchronization procedure with the external device and one or more auxiliary clocks used for other IMD operations. These other clocks could then run asynchronous with the external device and the synchronization clock. Alternatively, they can separately and internally be synchronized with the synchronization clock.

Note that in most practical implementation the IMD 100 will typical have the slave role in the synchronization. However, in some instances the IMD 100 may actually be the master with the external device as the slave. A clock manager 130 in the IMD 100 sets the IMD clock 120 upon reception of a synchronization set-up message from an external device. Furthermore, the manager 130 also provides a reference time to be returned to the external device. In the case, where the IMD 100 has the master role, the clock manager 130 retrieves the reference time to be included in the set-up message from the clock 120 and forwards it to the I/O unit 110 that transmits the message and reference time to the slave-acting external device. When receiving an ACK response from this external device, the I/O unit 110 forwards the reference time included therein to the synchronization unit 170 of the IMD 100. This unit 170 basically operates similar to the synchronization unit of the master device described above in connection with Fig. 18 and is not further discussed herein.

The units 110, 120, 130, 140, 160 and 170 of the IMD 100 may be provided as hardware, software or a combination of hardware and software. The units 110 to 170 can all be implemented together in the IMD 100. Alternatively, the IMD 100 could be segmented into a dedicated synchronization unit including the functionality of the units 120, 130 and 170, a communications module including the functionality of the units 110, 112 and 116, and a data acquisition unit including the functionality of the units 110, 114, 140, 150 and 160.

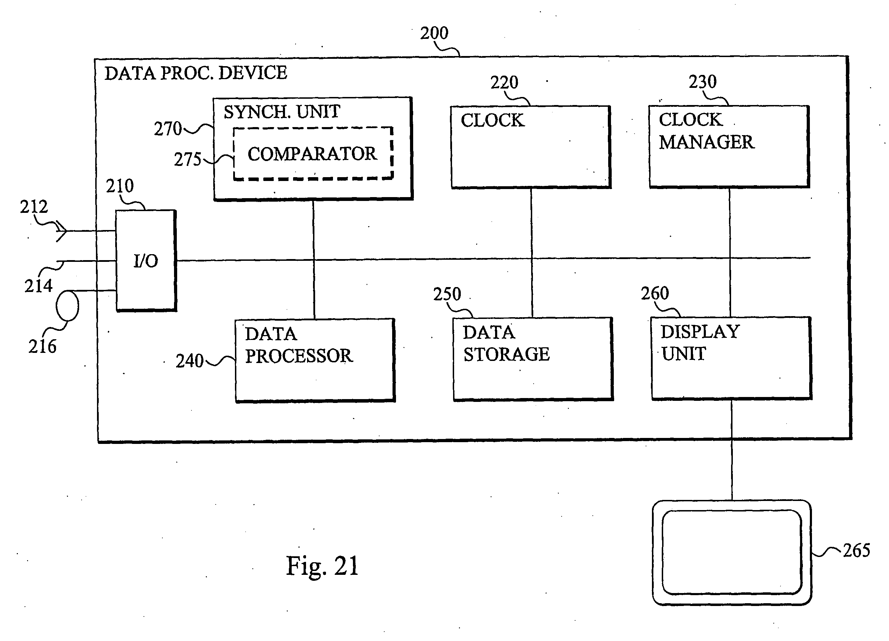

Fig. 21 is a schematic block diagram of a data processing device 200 according to the present invention. The data processing device 200 includes an I/O unit 210 for conducting wireless communication with at least one IMD and possibly wired or wireless communication with an external non-implanted diagnostic device. This I/O unit 210 includes functionalities for processing incoming and outgoing data messages, including modulator/demodulator and coder/decoder. The I/O unit 210 is further preferably connected to an antenna arrangement 212 used for transmitting and receiving radio packets to and from the IMD and optionally an external diagnostic device, respectively. However, the I/O unit 210 could also or alternatively use other forms of wireless techniques than radio packets for communication. The I/O unit 210 could for example use an inductive antenna 216 for external wireless communication. The I/O unit 210 can also be connected directly to one or more external diagnostic devices using the schematically illustrated wire 214.

The data processing device 200 further includes a data processor 240 that is used for processing physiological and diagnostic input data from the IMD and any external diagnostic devices. This processor 240 preferably uses time-information included in the physiological data or data blocks received by the I/O unit 210 in the data processing. For example, if the IMD transmits its sampled physiological data in the form of a number of time-stamped radio packets, the data processor 240 can use this time information for compiling and organizing the received data in the order it was sampled by the IMD. This will be very important in the case a particular radio packet had to be re-transmitted several times by the IMD before it was correctly received and decoded by the I/O unit 210. In such a case, the data processor 240 can use the time data included in the radio packet to correctly insert the data into the previously received data stream. Furthermore, in the case of any missing data, because e.g. too many retransmission so the data is no longer relevant, the data processor 240 can use the time-stamping for compensating for time gaps due to the missing data.

In the case the I/O unit 210 receives physiological data from multiple sources, e.g. the IMD and a surface ECG recorder, the data processor 240 can use the time-stamping for co-processing the different data. Thus, using the time-stamping the data processor 240 can provide time relationships between the IMD generated data and the surface ECG data.

The data processed by the processor 240 can be, at least, temporarily stored in a data storage 250 in the data processing device 200, or forwarded using the I/O unit 210 to an external storage. A clinician may later retrieve the physiological data from the storage 250 and use it for investigating the operation of the IMD and the physiological condition of the relevant patient or subject. The data stored in the storage 250 preferably still includes its time-stamping so that the clinician or the data processor 240 later can elucidate when it was actually sampled by the IMD or external diagnostic device.

In addition, or alternatively, to providing the (co-)processed data to the data storage 250, the data processor 240 can forward it to a display unit 260 that displays it on an associated display screen 265. Due to the time-stamping, the change in a measured physiological parameter can be viewed over time on the screen 265. In the case of multiple different physiological data, possibly from different sources, the display unit 260 can co-display this data on the screen 265. In such a case, the change in multiple physiological parameters over time can be viewed together on the single screen 265. For example, the screen 265 can together display an IEGM derived from IMD-based measurements and a surface ECG and possibly IMD-collected event markers.

In addition to the units 240-265 used for data management, the data processing device 200 preferably includes a clock 220 and clock manager 230. The processing device 200 further preferably comprises a synchronization unit 270, which may be used together with the clock 220 and clock manager 230 in a synchronization procedure involving the processing device 200 and an IMD or the processing device 200 and an external diagnostic device. The operation and function of these units 220, 230 and 270 basically correspond to the corresponding units in Figs. 18 and 19 and is not repeated herein.

The units 210, 220, 230, 240, 260, 270 and 275 of the data processing device 200 may be provided as hardware, software or a combination of hardware and software. The units 210 to 270 can all be implemented together in the data processing device 200. Alternatively, the data processing device 200 could include only those units 240 to 265 used for data management in addition to the I/O unit 210. In such a case, a separate external synchronization device or programmer could participate and manage the synchronization procedure of the invention with IMDs. This dedicated synchronization device could then include the units 220, 230 and 270 in addition to an I/O unit 210.

The data processing device can be connected to or communicating with a stand-alone external diagnostic device as was discussed above. In an alternative implementation, this diagnostic data measuring or recording functionality could be included in and constituting a part of the data processing device.

Return briefly to the synchronization and data processing system 1 of Fig. 1. In a preferred embodiment of the invention, the IMD 100 and the data processing device 200 is firstly synchronized with each other before communicating any physiological or operation data therebetween. For example, the data processing device 200 can initiate the synchronization according to the present invention by providing a current reference time from its internal clock. This reference time is included in a synchronization set-up message that is communicated to the IMD 100 over the link 190. The IMD 100 sets its internal clock based on the reference time in the received message. The IMD 100 further retrieves a current reference time from its adjusted clock and inserts it in an ACK response that is returned to the data processing device 200 over the link 190. If the reference time in the ACK response is associated with the time interval during which the processing device 200 received the ACK response, the two devices 100, 200 are regarded as synchronized.

The IMD 100 can now start sampling data, e.g. using the lead and sensor inserted in the patient's heart 15. As the data is sampled, or as it is transmitted, preferably in the form of radio packets, the IMD 100 time-stamps the data and/or the radio packets using the synchronized IMD clock. The data processing device 200 can then process the received and time-stamped physiological data from the IMD 100 by e.g. presenting it on the display scrren 265.

A same synchronization procedure can also be conducted involving the data processing device 200 and the ECG recorder 300, allowing a synchronization of all devices 100, 200, 300 in the system 1. In such a case, both the IMD 100 and the ECG recorder 300 preferably time-stamps their respective collected physiological data. The data processing device 200 can use the time-stamping for co-processing, e.g. co-displaying, the data from the two units 100, 300.

Note that the system 1 displayed in Fig. 1 could, as was briefly mentioned above, include a dedicated external synchronization device that manages the synchronization of the IMD 100 and optionally the ECG recorder 300. Once these devices 100, 300 has been synchronized with the synchronization device, they can start collecting and time-stamping data that is transmitted to the data processing device 200 for further processing and/or display. In such an embodiment, the data processing device 200 only includes data management functionality and the synchronization functionality is housed within the synchronization device.

It will be understood by a person skilled in the art that various modifications and changes may be made to the present invention without departure from the scope thereof, which is defined by the appended claims.

**Claims**

1. A method of synchronizing an implantable medical device (100) and an external device (200; 300) comprising:

   a master (400), being selected from one of said implantable medical device (100) and said external device (200; 300), communicating, in a first time interval ($T_k$), a synchronization set-up message to a slave (500), being selected from the other one of said implantable medical device (100) and said external device (200; 300);
   said master (400) receiving, in a second time interval ($T_{k+h}$) and from said slave (500), an acknowledge response, wherein
   said external device (200; 300) is an external non-implanted device (200; 300);
   said master (400) communicating being said master (400) wirelessly communicating, in said first time interval ($T_k$), said synchronization set-up message being in the form of a first reference time ($t_k$) associated with said first time interval ($T_k$) to said slave (500) over a wireless link (190);
   said master (400) receiving being said master (400) wirelessly receiving, in said second time interval ($T_{k+h}$) and from said slave (500) over said wireless link (190), said acknowledge response comprising a second reference

time ($t_{k+h}$) provided by said slave (500) based on said first reference time ($t_k$), said method further comprising:

said master (400) comparing said second reference time ($t_{k+h}$) with a current reference time associated with said second time interval ($T_{k+h}$) and obtained from a clock (420) associated with said master (400); and said master (400) confirming that said slave (500) is synchronized with said master (400) if said second reference time ($t_{k+h}$) corresponds to said current reference time.

2. A method of synchronizing an implantable medical device (100) and an external device (200; 300) comprising the steps of:

a slave (500), being selected from one of said implantable medical device (100) and said external device (200; 300), receiving, in a first time interval ($T_k$), a synchronization set-up message from a master (400), being selected from the other one of said implantable medical device (100) and said external device (200; 300); and said slave (500) communicating, in a second time interval ($T_{k+h}$) and to said master (400), an acknowledge response wherein

said external device (200; 300) is an external non-implanted device (200; 300);
said slave (500) receiving being said slave (500) wirelessly receiving, in said first time interval ($T_k$) and on a wireless link (190), said synchronization set-up message being in the form of a first reference time ($t_k$) associated with said first time interval ($T_k$);
said slave (500) communicating being said slave (500) wirelessly communicating, in said second time interval ($T_{k+h}$) and to said master (400) over said wireless link (190), said acknowledge response comprising a second reference time ($t_{k+h}$) associated with said second time interval ($T_{k+h}$) and provided by said slave (500) based on said first reference time ($t_k$), said method further comprising:

said slave (500) setting an associated clock (520) based on said first reference time ($t_k$), wherein said slave (500) and said master (400) are confirmedly synchronized provided that said acknowledge response is successfully received by said master (400) in said second time interval ($T_{k+h}$); said master comparing said second reference time ($t_{k+h}$) with a current reference time associated with said second time interval ($t_{k+h}$) and obtained from a clock (420) associated with said master (400); and said master (400) confirming that said slave (500) is synchronised with said master (400) if said second reference time ($t_{k+h}$) corresponds to said current reference time.

3. The method according to claim 1 or 2, **characterized by** one of said master (400) and said slave (500) communicating, to the other one of said master (400) and said slave (500), information of the length of a time interval ($T_k$, $T_{k+h}$) defined by an associated clock (420; 520).

4. The method according to any of the claims 1 to 3, **characterized in that** a reference time ($t_k$, $t_{k+h}$) being equal to a time indicative of a time interval ($T_k$, $T_{k+h}$) associated with said reference time ($t_k$, $t_{k+n}$) plus a predefined time delay ($\tau_1$, $\tau_2$).

5. A method of processing diagnostic data in an external non-implanted device (200) comprising the steps of:

synchronizing a clock (120) associated with an implantable medical device (100) with said external non-implanted device (200) according to the method of any of the claims 1 to 4;
said external non-implanted device (200) wirelessly receiving, from said implantable medical device (100) and over a wireless link (190), diagnostic data time-stamped using said associated clock (120); and said external device (200) processing said diagnostic data based on the time-stamp.

6. The method according to claim 5, **characterized by:**

synchronizing a clock associated with an external diagnostic device (300) with said external non-implanted device (200); and
said external non-implanted device (200) receiving, from said external diagnostic device (300), diagnostic data time-stamped using said associated clock of said external diagnostic device (300), said processing step comprises the step of said external non-implanted device (200) co-processing said diagnostic data from said implantable medical device (100) and said diagnostic data from said external diagnostic device (300) based on the time-stamps.

7. The method according to claim 5, **characterized in that** said external non-implanted device (200) comprises an internal diagnostic device (300), and said processing step comprises the step of said external non-implanted device (200) co-processing said diagnostic data from said implantable medical device (100) and diagnostic data from said internal diagnostic device based on the time-stamps.

8. A computer program product comprising software code for making a processor perform the steps of any of the claims 1 to 7.

9. A computer readable medium containing a computer program product according to claim 8.

10. A synchronization master device (400) being one of an implantable medical device (100) and an external device (200; 300), said synchronization master device (400) comprising:

a transmitter (410) for communicating, in a first time interval ($T_k$), a synchronization set-up message to a synchronization slave device (500), being selected from the other one of said implantable medical device (100) and said external device (200; 300);
a receiver (410) for receiving, in a second time interval ($T_{k+h}$) and from said synchronization slave device (500), an acknowledge response, wherein
said external device (200; 300) is an external non-implanted device (200; 300);
said transmitter (410) is configured for wirelessly communicating, in said first time interval ($T_k$), said synchronization set-up message being in the form of a first reference time ($t_k$) associated with said first time interval ($T_k$) to said synchronization slave device (500) over a wireless link (190);
said receiver (410) is configured for wirelessly receiving, in said second time interval ($T_{k+h}$) and from said synchronization slave device (500) over said wireless link (190), said acknowledge response comprising a second reference time ($t_{k+h}$) provided by said synchronization slave device (500) based on said first reference time ($t_k$), said synchronization master device (400) further comprises:

means (475) for comparing said second reference time ($t_{k+h}$) with a current reference time associated with said second time interval ($T_{k+h}$) and obtained from a clock (420) associated with said synchronization master device (400); and
means (470) for confirming that said synchronization slave device (500) is synchronized with said synchronization master device (400) if said second reference time ($t_{k+h}$) corresponds to said current reference time.

11. A system comprising a sychronization master device (1400) according to claim 10 and said synchronization slave device (500), said synchronization slave device (500) comprising:

a receiver (510) for receiving, in said first time interval ($T_k$), said synchronization set-up message from said synchronization master device (400), and
a transmitter (510) for communicating, in said second time interval ($T_{k+h}$) and to said synchronization master device (400), said acknowledge response, wherein
said receiver (510) is configured for wirelessly receiving, in said first time interval ($T_k$) and from said synchronization master device (400) on said wireless link (190);
said transmitter (510) is configured for wirelessly communicating, in said second time interval ($T_{k+h}$) and to said synchronization master device (400) over said wireless link (190), said acknowledge response comprising said second reference time ($t_{k+h}$) associated with said second time interval ($T_{k+h}$) and provided by said synchronization slave device (500) based on said first reference time ($t_k$), said synchronization slave device (500) further comprising:

means (530) for setting an associated clock (520) based on said first reference time ($t_k$), wherein said synchronization slave device (500) and said synchronization master device (400) are confirmedly synchronized provided that said acknowledge response is successfully received by said synchronization master device (400) in said second time interval ($T_{k+h}$).

12. The device according to claim 10 or 11, **characterized in that** one of said synchronization master device (400) and said synchronization slave device (500) is configured for communicating, to the other one of said synchronization master device (400) and said synchronization slave device (500), information of the length of a time interval ($T_k$, $T_{k+h}$) defined by an associated clock (420; 520).

**13.** The device according to any of the claims 10 to 12, **characterized in that** a reference time ($t_k$, $t_{k+h}$) being equal to a time indicative of a time interval ($T_k$, $T_{k+h}$) associated with said reference time ($t_k$, $t_{k+h}$) plus a predefined time delay ($\tau_1$, $\tau_2$).

**14.** The device according to any of the claims 10 to 13, **characterized in that** said synchronization master device (400) is a data acquisition system (200) and said synchronization slave device (500) is selected from a group consisting of a pacemaker, an implantable cardioverter and an implantable defibrillator (100).

**15.** A system (1) for processing diagnostic data in an external non-implanted device (200) comprising:

an implantable medical device (100) being one of a synchronization master device (400) according to claim 10 and a synchronization slave device (500) according to claim 11, wherein said implantable medical device (100) comprising an associated clock (120); and

an external non-implanted device (200) being the other of said synchronization master device (400) according to claim 10 and said synchronization slave device (500) according to claim 11, wherein said external non-implanted device (200) comprising:

a receiver (210) for wirelessly receiving, from said implantable medical device (100) and over a wireless link (190), diagnostic data time-stamped using said associated clock (120); and

a data processor (240) for processing said diagnostic data based on the time-stamp.

**16.** The system according to claim 15, **characterized in that** said system (1) is configured for synchronizing a clock associated with an external diagnostic device (300) with said external non-implanted device (200), said receiver (210) is configured for receiving, from said external diagnostic device (300), diagnostic data time-stamped using said associated clock of said external diagnostic device (300), and said data processor (240) is configured for co-processing said diagnostic data from said implantable medical device (100) and said diagnostic data from said external diagnostic device (300) based on the time-stamps.

**17.** The system according to claim 15, **characterized in that** said external non-implanted device (200) comprises an internal diagnostic device (300), and said data processor (240) is configured for co-processing said diagnostic data from said implantable medical device (100) and diagnostic data from said internal diagnostic device based on the time-stamps.

**Patentansprüche**

**1.** Verfahren zum Synchronisieren einer implantierbaren medizinischen Vorrichtung (100) und einer externen Vorrichtung (200; 300), umfassend:

Kommunizieren, durch einen Master (400), der aus einer der implantierbaren medizinischen Vorrichtung (100) und der externen Vorrichtung (200; 300) ausgewählt wird, einer Synchronisationsaufbaunachricht in einem ersten Zeitintervall ($T_k$) zu einem Slave (500), der aus der anderen der implantierbaren medizinischen Vorrichtung (100) und der externen Vorrichtung (200; 300) ausgewählt wird;
Empfangen, durch den Master (400), einer Bestätigungsantwort in einem zweiten Zeitintervall ($T_{k+h}$) und von dem Slave (500), wobei
die externe Vorrichtung (200; 300) eine externe nicht-implantierte Vorrichtung (200; 300) ist;
Kommunizieren, durch den Master (400), der der Master (400) ist, der drahtlos kommuniziert, der Synchronisationsaufbaunachricht, die in der Form einer ersten Referenzzeit ($t_k$) ist, die dem ersten Zeitintervall ($T_k$) zugeordnet ist, zu dem Slave (500) über eine drahtlose Verbindung (190) in dem ersten Zeitintervall ($T_k$);
Empfangen, durch den Master (400), der der Master (400) ist, der drahtlos empfängt, der Bestätigungsnachricht, die eine zweite Referenzzeit ($t_{k+h}$) umfasst, die von dem Slave (500) auf Grundlage der ersten Referenzzeit ($t_k$) bereitgestellt wird, in dem zweiten Zeitintervall ($T_{k+h}$) und von dem Slave (500) über die drahtlose Verbindung (190), wobei das Verfahren ferner umfasst:

Vergleichen, durch den Master (400), der zweiten Referenzzeit ($t_{k+h}$) mit einer gegenwärtigen Referenzzeit, die dem zweiten Zeitintervall ($T_{k+h}$) zugeordnet ist und von einer Uhr (420) erhalten wird, die dem Master (400) zugeordnet ist; und
Bestätigen, durch den Master (400), dass der Slave (500) mit dem Master (400) synchronisiert ist, wenn

die zweite Referenzzeit ($t_{k+h}$) der gegenwärtigen Referenzzeit entspricht.

2. Verfahren zum Synchronisieren einer implantierbaren medizinischen Vorrichtung (100) und einer externen Vorrichtung (200; 300), umfassend die Schritte:

> Empfangen, durch einen Slave (500), der aus einer der implantierbaren medizinische Vorrichtung (100) und der externen Vorrichtung (200; 300) ausgewählt wird, einer Synchronisationsaufbaunachricht von einem Master (400), der aus der anderen der implantierbaren medizinischen Vorrichtung (100) und der externen Vorrichtung (200; 300) ausgewählt wird, in einem ersten Zeitintervall ($T_k$); und
> Kommunizieren, durch den Slave (500), einer Bestätigungsantwort in einem zweiten Zeitintervall ($T_{k+h}$) und zu dem Master (400), wobei
> die externe Vorrichtung (200; 300) eine externe nicht-implantierte Vorrichtung (200; 300) ist;
> Empfangen, durch den Slave (500), der der Slave (500) ist, der drahtlos empfängt, der Synchronisationsaufbaunachricht, die in der Form einer ersten Referenzzeit ($t_k$) ist, die dem ersten Zeitintervall ($T_k$) zugeordnet ist, in dem ersten Zeitintervall ($T_k$) und über eine drahtlose Verbindung (190);
> Kommunizieren, durch den Slave (500), der der Slave (500) ist, der drahtlos kommuniziert, der Bestätigungsantwort, die eine zweite Referenzzeit ($t_{k+h}$) umfasst, die dem zweiten Zeitintervall ($T_{k+h}$) zugeordnet ist und von dem Slave (500) auf Grundlage der ersten Referenzzeit ($t_k$) bereitgestellt wird, in dem zweiten Zeitintervall ($T_{k+h}$) und zu dem Master (400) über die drahtlose Verbindung (190), wobei das Verfahren ferner umfasst:
>
>> Einstellen, durch den Slave (500), einer zugeordneten Uhr (520) auf Grundlage der ersten Referenzzeit ($t_k$), wobei der Slave (500) und der Master (400) abgesichert synchronisiert werden, vorausgesetzt, dass die Bestätigungsantwort von dem Master (400) in dem zweiten Zeitintervall ($T_{k+h}$) erfolgreich empfangen wird;
>> Vergleichen, durch den Master, der zweiten Referenzzeit ($t_{k+k}$) mit einer gegenwärtigen Referenzzeit, die dem zweiten Zeitintervall ($T_{k+h}$) zugeordnet ist und von einer Uhr (420) erhalten wird, die dem Master (400) zugeordnet ist; und
>> Bestätigen, durch den Master (400), dass der Slave (500) mit dem Master (400) synchronisiert ist, wenn die zweite Referenzzeit ($t_{k+h}$) der gegenwärtigen Referenzzeit entspricht.

3. Verfahren nach Anspruch 1 oder 2, **dadurch gekennzeichnet, dass** einer von dem Master (400) und dem Slave (500) zu dem anderen von dem Master (400) und dem Slave (500) eine Information der Länge eines Zeitintervalls ($T_k$, $T_{k+h}$) kommuniziert, das durch eine zugeordnete Uhr (420; 520) definiert ist.

4. Verfahren nach einem der Ansprüche 1 bis 3, **dadurch gekennzeichnet, dass** eine Referenzzeit ($t_k$, $t_{k+h}$) gleich einer Zeit ist, die indikativ für ein Zeitintervall ($T_k$, $T_{k+h}$) ist, das der Referenzzeit ($t_k$, $t_{k+h}$) plus einer vordefinierten Zeitverzögerung ($T_1$, $T_2$) zugeordnet ist.

5. Verfahren zum Verarbeiten von Diagnostikdaten in einer externen nicht-implantierten Vorrichtung (200), umfassend die Schritte:

> Synchronisieren einer Uhr (120), die einer implantierbaren medizinischen Vorrichtung (100) zugeordnet ist, mit der externen nicht-implantierten Vorrichtung (200) gemäß dem Verfahren nach einem der Ansprüche 1 bis 4;
> wobei die externe nicht-implantierte Vorrichtung (200) von der implantierbaren medizinischen Vorrichtung (100) und über eine drahtlose Verbindung (190) Diagnostikdaten drahtlos empfängt, die unter Verwendung der zugeordneten Uhr (120) mit einem Zeitstempel versehen sind; und
> wobei die externe Vorrichtung (200) die Diagnostikdaten auf Grundlage des Zeitstempels verarbeitet.

6. Verfahren nach Anspruch 5, **gekennzeichnet durch:**

> Synchronisieren einer Uhr, die einer externen Diagnostikvorrichtung (300) zugeordnet ist, mit der externen nicht-implantierten Vorrichtung (200); und
> Empfangen, **durch** die externe nicht-implantierte Vorrichtung (200), von Diagnostikdaten, die unter Verwendung der zugeordneten Uhr der externen Diagnostikvorrichtung (300) mit einem Zeitstempel versehen sind, von der externen Diagnostikvorrichtung (300), wobei der Verarbeitungsschritt den Schritt umfasst, dass die externe nicht-implantierte Vorrichtung (200) die Diagnostikdaten von der implantierbaren medizinischen Vorrichtung (100) und die Diagnostikdaten von der externen Diagnostikvorrichtung (300) auf Grundlage der Zeitstempel gemeinsam verarbeitet.

7. Verfahren nach Anspruch 5, **dadurch gekennzeichnet, dass** die externe nicht-implantierte Vorrichtung (200) eine interne Diagnostikvorrichtung (300) umfasst, und der Verarbeitungsschritt den Schritt umfasst, dass die externe nicht-implantierte Vorrichtung (200) die Diagnostikdaten von der implantierbaren medizinischen Vorrichtung (100) und Diagnostikdaten von der internen Diagnostikvorrichtung auf Grundlage der Zeitstempel gemeinsam verarbeitet.

8. Computerprogrammprodukt, umfassend einen Software-Code, der bewirkt, dass ein Prozessor die Schritte nach einem der Ansprüche 1 bis 7 durchführt.

9. Computerlesbares Medium, das ein Computerprogrammprodukt gemäß Anspruch 8 enthält.

10. Synchronisations-Master-Vorrichtung (400), die eine einer implantierbaren medizinischen Vorrichtung (100) und einer externen Vorrichtung (200; 300) ist, wobei die Synchronisations-Master-Vorrichtung (400) umfasst:

   einen Sender (410) zum Kommunizieren, in einem ersten Zeitintervall ($T_k$), einer Synchronisationsaufbaunachricht zu einer Synchronisations-Slave-Vorrichtung (500), die aus der anderen der implantierbaren medizinischen Vorrichtung (100) und der externen Vorrichtung (200; 300) ausgewählt ist;
   einen Empfänger (410) zum Empfangen, in einem zweiten Zeitintervall ($T_{k+h}$) und von der Synchronisations-Slave-Vorrichtung (500), einer Bestätigungsantwort, wobei
   die externe Vorrichtung (200; 300) eine externe nicht-implantierte Vorrichtung (200; 300) ist;
   der Sender (410) konfiguriert ist zum drahtlosen Kommunizieren der Synchronisationsaufbaunachricht, die in der Form einer ersten Referenzzeit ($t_k$) ist, die dem ersten Zeitintervall ($T_k$) zugeordnet ist, zu der Synchronisations-Slave-Vorrichtung (500) über eine drahtlose Verbindung (190) in dem ersten Zeitintervall ($T_k$);
   der Empfänger (410) konfiguriert ist zum drahtlosen Empfangen der Bestätigungsantwort, die eine zweite Referenzzeit ($t_{k+h}$) umfasst, die von der Synchronisations-Slave-Vorrichtung (500) auf Grundlage der ersten Referenzzeit ($t_k$) bereitgestellt ist, in dem zweiten Zeitintervall ($T_{k+h}$) und von der Synchronisations-Slave-Vorrichtung (500) über die drahtlose Verbindung (190), wobei die Synchronisations-Master-Vorrichtung (400) ferner umfasst:

   eine Einrichtung (475) zum Vergleichen der zweiten Referenzzeit ($t_{k+h}$) mit einer gegenwärtigen Referenzzeit, die dem zweiten Zeitintervall ($T_{k+h}$) zugeordnet ist und von einer Uhr (420), die der Synchronisations-Master-Vorrichtung (400) zugeordnet ist, erhalten wird; und
   eine Einrichtung (470) zum Bestätigen, dass die Synchronisations-Slave-Vorrichtung (500) mit der Synchronisations-Master-Vorrichtung (400) synchronisiert ist, wenn die zweite Referenzzeit ($t_{k+h}$) der gegenwärtigen Referenzzeit entspricht.

11. System, umfassend eine Synchronisations-Master-Vorrichtung (400) nach Anspruch 10 und die Synchronisations-Slave-Vorrichtung (500), wobei die Synchronisations-Slave-Vorrichtung (500) umfasst:

   einen Empfänger (510) zum Empfangen der Synchronisationsaufbaunachricht von der Synchronisations-Master-Vorrichtung (400) in dem ersten Zeitintervall ($T_k$); und
   einen Sender (510) zum Kommunizieren der Bestätigungsantwort in dem zweiten Zeitintervall ($T_{k+h}$) und zu der Synchronisations-Master-Vorrichtung (400), wobei
   der Empfänger (510) konfiguriert ist zum drahtlosen Empfangen, in dem ersten Zeitintervall ($T_k$) und von der Synchronisations-Master-Vorrichtung (400), über die drahtlose Verbindung (190);
   der Sender (510) konfiguriert ist zum drahtlosen Kommunizieren, in dem zweiten Zeitintervall ($T_{k+h}$) und zu der Synchronisations-Master-Vorrichtung (400) über die drahtlose Verbindung (190), der Bestätigungsantwort, die die zweite Referenzzeit ($t_{k+h}$) umfasst, die dem zweiten Zeitintervall ($T_{k+h}$) zugeordnet ist und von der Synchronisations-Slave-Vorrichtung (500) auf Grundlage der ersten Referenzzeit ($t_k$) bereitgestellt ist, wobei die Synchronisations-Slave-Vorrichtung (500) ferner umfasst:

   eine Einrichtung (530) zum Einstellen einer zugeordneten Uhr (520) auf Grundlage der ersten Referenzzeit ($t_k$), wobei die Synchronisations-Slave-Vorrichtung (500) und die Synchronisations-Master-Vorrichtung (400) abgesichert synchronisiert werden, vorausgesetzt, dass die Bestätigungsantwort von der Synchronisations-Master-Vorrichtung (400) in dem zweiten Zeitintervall ($T_{k+h}$) erfolgreich empfangen wird.

12. Vorrichtung nach Anspruch 10 oder 11, **dadurch gekennzeichnet, dass** eine der Synchronisations-Master-Vorrichtung (400) und der Synchronisations-Slave-Vorrichtung (500) konfiguriert ist zum Kommunizieren von Information der Länge eines Zeitintervalls ($T_k$, $T_{k+h}$), das durch eine zugeordnete Uhr (420; 520) definiert ist, zu der anderen der Synchronisations-Master-Vorrichtung (400) und der Synchronisations-Slave-Vorrichtung (500).

13. Vorrichtung nach einem der Ansprüche 10 bis 12, **dadurch gekennzeichnet, dass** eine Referenzzeit ($t_k$, $t_{k+h}$) gleich einer Zeit ist, die indikativ für ein Zeitintervall ($T_k$, $T_{k+h}$) ist, das der Referenzzeit ($t_k$, $t_{k+h}$) plus einer vordefinierten Zeitverzögerung ($T_1$, $T_2$) zugeordnet ist.

14. Vorrichtung nach einem der Ansprüche 10 bis 13, **dadurch gekennzeichnet, dass** die Synchronisations-Master-Vorrichtung ein Datenerfassungssystem (200) ist und die Synchronisations-Slave-Vorrichtung (500) aus einer Gruppe gewählt ist, die aus einem Schrittmacher, einem implantierbaren Kardioverter und einem implantierbaren Defibrillator (100) besteht.

15. System (1) zum Verarbeiten von Diagnostikdaten in einer externen nicht-implantierten Vorrichtung (200), umfassend:

eine implantierbare medizinische Vorrichtung (100), die eine einer Synchronisations-Master-Vorrichtung (400) nach Anspruch 10 und einer Synchronisations-Slave-Vorrichtung (500) nach Anspruch 11 ist, wobei die implantierbare medizinische Vorrichtung (100) eine zugeordnete Uhr (120) umfasst, und
wobei eine externe nicht-implantierte Vorrichtung (200) die andere der Synchronisations-Master-Vorrichtung (400) nach Anspruch 10 und der Synchronisations-Slave-Vorrichtung (500) nach Anspruch 11 ist, wobei die externe nicht-implantierte Vorrichtung (200) umfasst:

einen Empfänger (210) zum drahtlosen Empfangen, von der implantierbaren medizinischen Vorrichtung (100) und über eine drahtlose Verbindung (190), von Diagnostikdaten, die unter Verwendung der zugeordneten Uhr (120) mit einem Zeitstempel versehen sind; und
einen Datenprozessor (240) zum Verarbeiten der Diagnostikdaten auf Grundlage des Zeitstempels.

16. System nach Anspruch 15, **dadurch gekennzeichnet, dass** das System (1) konfiguriert ist zum Synchronisieren einer Uhr, die einer externen Diagnostikvorrichtung (300) zugeordnet ist, mit der externen nicht-implantierten Vorrichtung (200), wobei der Empfänger (210) konfiguriert ist zum Empfangen von Diagnostikdaten, die unter Verwendung der zugeordneten Uhr der externen Diagnostikvorrichtung (300) mit einem Zeitstempel versehen sind, von der externen Diagnostikvorrichtung (300), und der Datenprozessor (240) konfiguriert ist zum gemeinsamen Verarbeiten der Diagnostikdaten von der implantierbaren medizinischen Vorrichtung (100) und der Diagnostikdaten von der externen Diagnostikvorrichtung (300) auf Grundlage der Zeitstempel.

17. System nach Anspruch 15, **dadurch gekennzeichnet, dass** die externe nicht-implantierte Vorrichtung (200) eine interne Diagnostikvorrichtung (300) umfasst, und der Datenprozessor (240) zum gemeinsamen Verarbeiten der Diagnostikdaten von der implantierbaren medizinischen Vorrichtung (100) und Diagnostikdaten von der internen Diagnostikvorrichtung auf Grundlage der Zeitstempel konfiguriert ist.

**Revendications**

1. Procédé de synchronisation d'un dispositif médical implantable (100) et d'un dispositif externe (200 ; 300) comprenant :

un maître (400), sélectionné parmi l'un des dispositifs incluant ledit dispositif médical implantable (100) et ledit dispositif externe (200 ; 300), communiquant, dans un premier intervalle de temps ($T_k$), un message d'établissement de synchronisation à un esclave (500), sélectionné parmi l'autre des dispositifs incluant ledit dispositif médical implantable (100) et ledit dispositif externe (200 ; 300) ;
ledit maître (400) recevant, dans un deuxième intervalle de temps ($T_{k+h}$) et à partir dudit esclave (500), une réponse d'accusé de réception, dans lequel
ledit dispositif externe (200 ; 300) est un dispositif externe non-implanté (200 ; 300) ;
ledit maître (400) communiquant étant ledit maître (400) communiquant sans fil, dans ledit premier intervalle de temps ($T_k$), ledit message d'établissement de synchronisation étant sous la forme d'un premier temps de référence ($t_k$) associé audit premier intervalle de temps ($T_k$) audit esclave (500) sur une liaison sans fil (190) ;
ledit maître (400) recevant étant ledit maître (400) recevant sans fil, dans ledit deuxième intervalle de temps ($T_{k+h}$) et à partir dudit esclave (500) sur ladite liaison sans fil (190), ladite réponse d'accusé de réception comprenant un deuxième temps de référence ($t_{k+h}$) fourni par ledit esclave (500) sur la base dudit premier temps de référence ($t_k$), ledit procédé comprenant en outre :

ledit maître (400) comparant ledit deuxième temps de référence ($t_{k+h}$) à un temps de référence actuel associé audit deuxième intervalle de temps ($T_{k+h}$) et obtenu à partir d'une horloge (420) associée audit maître (400) ; et

ledit maître (400) confirmant que ledit esclave (500) est synchronisé avec ledit maître (400) si ledit deuxième temps de référence ($t_{k+h}$) correspond audit temps de référence actuel.

2. Procédé de synchronisation d'un dispositif médical implantable (100) et d'un dispositif externe (200 ; 300) comprenant les étapes suivantes :

un esclave (500), sélectionné parmi l'un des dispositifs incluant ledit dispositif médical implantable (100) et ledit dispositif externe (200 ; 300), recevant, dans un premier intervalle de temps ($T_k$), un message d'établissement de synchronisation à partir d'un maître (400), sélectionné parmi l'autre des dispositifs incluant ledit dispositif médical implantable (100) et ledit dispositif externe (200 ; 300) ; et

ledit esclave (500) communiquant, dans un deuxième intervalle de temps ($T_{k+h}$) et audit maître (500), une réponse d'accusé de réception, dans lequel

ledit dispositif externe (200 ; 300) est un dispositif externe non-implanté (200 ; 300) ;

ledit esclave (500) recevant étant ledit esclave (500) recevant sans fil, dans ledit premier intervalle de temps ($T_k$) et sur une liaison sans fil (190), ledit message d'établissement de synchronisation étant sous la forme d'un premier temps de référence ($t_k$) associé audit premier intervalle de temps ($T_k$) ;

ledit esclave (500) communiquant étant ledit esclave (500) communiquant sans fil, dans ledit deuxième intervalle de temps ($T_{k+h}$) et audit maître (400) sur ladite liaison sans fil (190), ladite réponse d'accusé de réception comprenant un deuxième temps de référence ($t_{k+h}$) associé audit deuxième intervalle de temps ($T_{k+h}$) et fourni par ledit esclave (500) sur la base dudit premier temps de référence ($t_k$), ledit procédé comprenant en outre :

ledit esclave (500) réglant une horloge (520) associée sur la base dudit premier temps de référence ($t_k$), dans lequel ledit esclave (500) et ledit maître (400) sont synchronisés de manière confirmée à condition que ladite réponse d'accusé de réception soit reçue avec succès par ledit maître (400) dans ledit deuxième intervalle de temps ($T_{k+h}$) ; ledit maître comparant ledit deuxième temps de référence ($t_{k+h}$) à un temps de référence actuel associé audit deuxième intervalle de temps ($T_{k+h}$) et obtenu à partir d'une horloge (420) associée audit maître (400) ; et ledit maître (400) confirmant que ledit esclave (500) est synchronisé avec ledit maître (400) si ledit deuxième temps de référence ($t_{k+h}$) correspond audit temps de référence actuel.

3. Procédé selon la revendication 1 ou 2, **caractérisé en ce qu'**un dispositif parmi ledit maître (400) et ledit esclave (500) communique, à l'autre dispositif parmi ledit maître (400) et ledit esclave (500), des informations de la longueur d'un intervalle de temps ($T_k$, $T_{k+h}$) défini par une horloge (420 ; 520) associée.

4. Procédé selon l'une quelconque des revendications 1 à 3, **caractérisé en ce qu'**un temps de référence ($t_k$, $t_{k+h}$) est égal à un temps indiquant un intervalle de temps ($T_k$, $T_{k+h}$) associé audit temps de référence ($t_k$, $t_{k+h}$) plus un temps de retard prédéfini ($\tau_1$, $\tau_2$).

5. Procédé de traitement de données de diagnostic dans un dispositif externe non-implanté (200) comprenant les étapes suivantes :

synchroniser une horloge (120) associée à un dispositif médical implantable (100) avec ledit dispositif externe non-implanté (200) conformément au procédé selon l'une quelconque des revendications 1 à 4 ;

ledit dispositif externe non-implanté (200) recevant sans fil, à partir dudit dispositif médical implantable (100) et sur une liaison sans fil (190), des données de diagnostic horodatées en utilisant ladite horloge (120) associée ; et

ledit dispositif externe (200) traitant lesdites données de diagnostic sur la base de la marque d'horodatage.

6. Procédé selon la revendication 5, **caractérisé par :**

la synchronisation d'une horloge associée à un dispositif externe de diagnostic (300) avec ledit dispositif externe non-implanté (200) ; et

la réception par ledit dispositif externe non-implanté (200), à partir dudit dispositif externe de diagnostic (300), de données de diagnostic horodatées en utilisant ladite horloge associée dudit dispositif externe de diagnostic (300), ladite étape de traitement comprend l'étape de co-traitement par ledit dispositif externe non-implanté (200) desdites données de diagnostic à partir dudit dispositif médical implantable (100) et desdites données de diagnostic à partir dudit dispositif externe de diagnostic (300) sur la base des marques d'horodatage.

7. Procédé selon la revendication 5, **caractérisé en ce que** ledit dispositif externe non-implanté (200) comprend un dispositif interne de diagnostic (300), et ladite étape de traitement comprend l'étape de co-traitement par ledit dispositif externe non-implanté (200) desdites données de diagnostic à partir dudit dispositif médical implantable (100) et desdites données de diagnostic à partir dudit dispositif interne de diagnostic sur la base des marques d'horodatage.

8. Produit de programme informatique comprenant un code logiciel pour qu'un processeur exécute les étapes selon l'une quelconque des revendications 1 à 7.

9. Support lisible par un ordinateur contenant un produit de programme informatique selon la revendication 8.

10. Dispositif maître de synchronisation (400) étant un dispositif parmi un dispositif médical implantable (100) et un dispositif externe (200 ; 300), ledit dispositif maître de synchronisation (400) comprenant :

un émetteur (410) pour communiquer, dans un premier intervalle de temps ($T_k$), un message d'établissement de synchronisation à un dispositif esclave de synchronisation (500), étant sélectionné parmi l'autre des dispositifs incluant ledit dispositif médical implantable (100) et ledit dispositif externe (200 ; 300) ;
un récepteur (410) pour recevoir, dans un deuxième intervalle de temps ($T_{k+h}$) et à partir dudit dispositif esclave de synchronisation (500), une réponse d'accusé de réception, dans lequel
ledit dispositif externe (200 ; 300) est un dispositif externe non-implanté (200 ; 300) ;
ledit émetteur (410) est configuré pour communiquer sans fil, dans ledit premier intervalle de temps ($T_k$), ledit message d'établissement de synchronisation étant sous la forme d'un premier temps de référence ($t_k$) associé audit premier intervalle de temps ($T_k$) audit dispositif esclave de synchronisation (500) sur une liaison sans fil (190) ;
ledit récepteur (410) est configuré pour recevoir sans fil, dans ledit deuxième intervalle de temps ($T_{k+h}$) et à partir dudit dispositif esclave de synchronisation (500) sur ladite liaison sans fil (190), ladite réponse d'accusé de réception comprenant un deuxième temps de référence ($t_{k+h}$) fourni par ledit dispositif esclave de synchronisation (500) sur la base dudit premier temps de référence ($t_k$), ledit dispositif maître de synchronisation (400) comprenant en outre :

un moyen (475) pour comparer ledit deuxième temps de référence ($t_{k+h}$) à un temps de référence actuel associé audit deuxième intervalle de temps ($T_{k+h}$) et obtenu à partir d'une horloge (420) associée audit dispositif maître de synchronisation (400) ; et
un moyen (470) pour confirmer que ledit dispositif esclave de synchronisation (500) est synchronisé avec ledit dispositif maître de synchronisation (400) si ledit deuxième temps de référence ($t_{k+h}$) correspond audit temps de référence actuel.

11. Système comprenant un dispositif maître de synchronisation (400) selon la revendication 10 et ledit dispositif esclave de synchronisation (500), ledit dispositif esclave de synchronisation (500) comprenant :

un récepteur (510) pour recevoir, dans ledit premier intervalle de temps ($T_k$), ledit message d'établissement de synchronisation à partir dudit dispositif maître de synchronisation (400) ; et
un émetteur (510) pour communiquer, dans ledit deuxième intervalle de temps ($T_{k+h}$) et audit dispositif maître de synchronisation (400), ladite réponse d'accusé de réception, dans lequel,
ledit récepteur (510) est configuré pour recevoir sans fil, dans ledit premier intervalle de temps ($T_k$) et à partir dudit dispositif maître de synchronisation (400) sur ladite liaison sans fil (190) ;
ledit émetteur (510) est configuré pour communiquer sans fil, dans ledit deuxième intervalle de temps ($T_{k+h}$) et audit dispositif maître de synchronisation (400) sur ladite liaison sans fil (190), ladite réponse d'accusé de réception comprenant ledit deuxième temps de référence ($t_{k+h}$) associé audit deuxième intervalle de temps ($T_{k+h}$) et fourni par ledit dispositif esclave de synchronisation (500) sur la base dudit premier temps de référence ($t_k$), ledit dispositif esclave de synchronisation (500) comprenant en outre :

un moyen (530) pour régler une horloge (520) associée sur la base dudit premier temps de référence ($t_k$), dans lequel ledit dispositif esclave de synchronisation (500) et ledit dispositif maître de synchronisation (400) sont synchronisés de manière confirmée à condition que ladite réponse d'accusé de réception soit reçue avec succès par ledit dispositif maître de synchronisation (400) dans ledit deuxième intervalle de

temps ($T_{k+h}$).

**12.** Dispositif selon la revendication 10 ou 11, **caractérisé en ce qu'**un dispositif parmi ledit dispositif maître de synchronisation (400) et ledit dispositif esclave de synchronisation (500) est configuré pour communiquer, à l'autre dispositif parmi ledit dispositif maître de synchronisation (400) et ledit dispositif esclave de synchronisation (500), des informations de la longueur d'un intervalle de temps ($T_k$, $T_{k+h}$) défini par une horloge (420 ; 520) associée.

**13.** Dispositif selon l'une quelconque des revendications 10 à 12, **caractérisé en ce qu'**un temps de référence ($t_k$, $t_{k+h}$) est égal à un temps indiquant un intervalle de temps ($T_k$, $T_{k+h}$) associé audit temps de référence ($t_k$, $t_{k+h}$) plus un temps de retard prédéfini ($\tau_1$, $\tau_2$).

**14.** Dispositif selon l'une quelconque des revendications 10 à 13, **caractérisé en ce que** ledit dispositif maître de synchronisation (400) est un système d'acquisition de données (200) et ledit dispositif esclave de synchronisation (500) est sélectionné dans un groupe constitué d'un stimulateur cardiaque, d'un défibrillateur à synchronisation automatique implantable et d'un défibrillateur implantable (100).

**15.** Système (1) pour traiter des données de diagnostic dans un dispositif externe non-implanté (200) comprenant :

un dispositif médical implantable (100) étant un dispositif parmi un dispositif maître de synchronisation (400) selon la revendication 10 et un dispositif esclave de synchronisation (500) selon la revendication 11, ledit dispositif médical implantable (100) comprenant une horloge (120) associée ; et
un dispositif externe non-implanté (200) étant l'autre dispositif parmi ledit dispositif maître de synchronisation (400) selon la revendication 10 et ledit dispositif esclave de synchronisation (500) selon la revendication 11, ledit dispositif externe non-implanté (200) comprenant :

un récepteur (210) pour recevoir sans fil, à partir dudit dispositif médical implantable (100) et sur une liaison sans fil (190), des données de diagnostic horodatées en utilisant ladite horloge (120) associée ; et
un dispositif de traitement de données (240) pour traiter lesdites données de diagnostic sur la base de la marque d'horodatage.

**16.** Système selon la revendication 15, **caractérisé en ce que** ledit système (1) est configuré pour synchroniser une horloge associée à un dispositif externe de diagnostic (300) avec ledit dispositif externe non-implanté (200), ledit récepteur (210) est configuré pour recevoir, à partir dudit dispositif externe de diagnostic (300), des données de diagnostic horodatées en utilisant ladite horloge associée dudit dispositif externe de diagnostic (300), et ledit dispositif de traitement de données (240) est configuré pour co-traiter lesdites données de diagnostic à partir dudit dispositif médical implantable (100) et lesdites données de diagnostic à partir dudit dispositif externe de diagnostic (300) sur la base des marques d'horodatage.

**17.** Système selon la revendication 15, **caractérisé en ce que** ledit dispositif externe non-implanté (200) comprend un dispositif interne de diagnostic (300), et ledit dispositif de traitement de données (240) est configuré pour co-traiter lesdites données de diagnostic à partir dudit dispositif médical implantable (100) et lesdites données de diagnostic à partir dudit dispositif interne de diagnostic sur la base des marques d'horodatage.

Fig. 1

EP 1 896 127 B1

Fig. 2

Fig. 3

Fig. 4

Fig. 5

Fig. 6

EP 1 896 127 B1

Fig. 7

Fig. 8

MASTER                    SLAVE

TIME INTERVAL LENGTH

$t_k$         SET REF. TIME $t_k$
$T_k$                                    REF. TIME $t_k$ SET

SYNCH.        $t_k$ ACK

$t_{k+1}$

$T_{k+1}$

$t_{k+2}$

TIME                    TIME      Fig. 9

MASTER                    SLAVE

$t_k$         SET REF. TIME $t_k$
$T_k$                                    REF. TIME $t_k$
                                         SET
SYNCH.        $t_k$ ACK

$t_{k+1}$

$T_{k+1}$

$t_{k+2}$

$\tau_1$  $t_{k+5}$

$T_{k+5}$  $\tau_2$    SET REF. TIME $t_{k+5}, \tau_1, \tau_2$    REF. TIME $t_{k+5}$
                                                                SET
SYNCH.        $t_{k+5}$ ACK
$t_{k+6}$

$T_{k+6}$

$t_{k+7}$

TIME                    TIME      Fig. 10

Fig. 11

Fig. 12

EP 1 896 127 B1

## Fig. 13

START

S1 — SEND 1ST REF. TIME IN 1ST TIME INT.

S2 — RECEIVE 2ND REF. TIME IN 2ND TIME INT.

S3 — CONFIRM SYNCH. IF 2ND REF. TIME IS ASSOCIATED WITH 2ND TIME INT.

L1

STOP

## Fig. 15

START

S20 — PROVIDE 1ST REF. TIME FROM CLOCK

S21 — PROVIDE TIME INT. LENGTH INFORMATION

S22 — SEND 1ST REF. TIME & LENGTH INFORMATION

TO STEP S2 OR S11

## Fig. 16

FROM STEP S2 OR S12

S30 — PROVIDE CURRENT REF. TIME

S31 — CURRENT REF. TIME = 2ND REF. TIME ?

YES

NO → TO STEP S1 OR S10

SYNCH. CONFIRMED — S32

STOP

```
          ┌─────────────┐
          │    START    │
          └─────────────┘
                 │
                 ▼                           ┌─ L2
          ┌─────────────────┐
   S10 ───│ SEND 1ST REF. TIME │
          │   IN 1ST TIME INT. │
          └─────────────────┘
                 │
                 ▼
          ┌─────────────────┐
   S11 ───│ SET CLOCK BASED │
          │  ON 1ST REF. TIME │
          └─────────────────┘
                 │
                 ▼
          ┌─────────────────┐
   S12 ───│  SEND 2ND REF.  │
          │      TIME       │
          └─────────────────┘
                 │
                 ▼
          ┌─────────────────┐
          │ CONFIRM SYNCH. IF │
   S13 ───│ 2ND REF. TIME IS │
          │ ASSOCIATED WITH │
          │   2ND TIME INT.  │
          └─────────────────┘
                 │
                 ▼
          ┌─────────────┐
          │    STOP     │
          └─────────────┘
```

Fig. 14

32

Fig. 17

Fig. 18

Fig. 19

EP 1 896 127 B1

Fig. 20

Fig. 21

EP 1 896 127 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030114898 A
- US 20040215272 A

- US 20040059396 A